# EUROPEAN PATENT SPECIFICATION

(11) **EP 2 118 065 B1**
(45) Date of publication and mention of the grant of the patent: **14.08.2013**
(21) Application number: 07849684.1
(22) Date of filing: 30.11.2007
(51) Int. Cl.: C07D 221/14, A61K 31/473, A61P 35/00

(54) **Substituted 1H-benz[de]isoquinoline-1,3-diones**
Substituierte 1H-benz[de]isochinolin-1,3-dione
1H-benz[de]isoquinoline-1,3-diones substitués

(30) Priority: 11.01.2007 IN DE00722007
(43) Date of publication of application: 18.11.2009
(73) Proprietor: Council of Scientific & Industrial Research, New Delhi 110 010 (IN); Chitaranjan National Cancer Institute, Kolkata 700 026 WB (IN)
(72) Inventor: QAZI, Ghulam, Nabi, Jammu Tawi 180 001 Jammu Kashmir (IN); SAXENA, Ajit, Kumar, Jammu Tawi 180 001 Jammu Kashmir (IN); MUTHIAH, Shanmugavel, Jammu Tawi 180 001 Jammu Kashmir (IN); MONDHE, Dilip, Manikrao, Jammu Tawi 180 001 Jammu Kashmir (IN); SHARMA, Parduman, Raj, Jammu Tawi 180 001 Jammu Kashmir (IN); SINGH, Shashank, Kumar, Jammu Tawi 180 001 Jammu Kashmir (IN); SANYAL, Utpal, Kolkata 700 026 West Bengal (IN); MUKHERJEE, Asama, Kolkata 700 026 West Bengal (IN); HAZRA, Suva, Kolkata 700 026 West Bengal (IN); DUTTA, Susanta, Kolkata 700 026 West Bengal (IN)
(74) Representative: Graf von Stosch, Andreas
(86) International application number: PCT/IN2007/000559
(87) International publication number: WO 2008/084496

(56) References cited:
- US-A- 3 940 397
- US-A- 4 077 958
- US-A1- 2004 082 788
- BRANA M F ET AL: "NAPHTHALIMIDES AS ANTI-CANCER AGENTS: SYNTHESIS AND BIOLOGICAL ACTIVITY" CURRENT MEDICINAL CHEMISTRY. ANTI-CANCER AGENTS, BENTHAM SCIENCE PUBLISHERS, HILVERSUM, NL, vol. 1, no. 3, November 2001 (2001-11), pages 237-255, XP008079357 ISSN: 1568-0118 cited in the application

## Description

### Field of the invention

The present invention relates to novel substituted 1*H*-benz[*de*]isoquinoline-1,3-diones. This invention particularly relates to novel substituted 1*H-*benz[*de*]isoquinoline-1,3-diones particularly N-[2-(Chloroethyl)]- and N-[3-(Chloropropyl)]-naphthalimides and antitumor activity thereof. The present invention also relates to process of preparation of novel substituted 1*H*-benz[*de*]isoquinoline-1,3-diones. The present invention further relates to pharmaceutical composition for the treatment of cancer.

### Background of the invention

N-(2,2-dimethylaminoethyl)naphthalimide derivative such as Mitonafide had exhibited¹ [Brana M.F. and Ramos A. (2001). Naphthalimides as anticancer agents: Synthesis and Biological Activity. Curr Med Chem - Anti-Cancer Agents 1: 237-255] interesting anti-tumoral activity in various experimental and clinical tumors.

In recent years ongoing research is focused towards the development of chemotherapeutic agents having properties like apoptosis [programmed cell death] etc other than the classical alkylating agents with a view to their plausible application in human cancers. Several structural patterns have been explored for this purpose. However, literature survey reveals that there are no reports, to our knowledge, in which the substituted naphthalimides have been used as such apoptotic agents. The naphthalimide ring was particularly chosen since it has been found that some substituted naphthalimides containing N-(2-dimethylaminoethyl) chain best represented by mitonafide possessing DNA binding properties, have exhibited substantial cytotoxicities in various animal and human tumors and have undergone clinical trials. Hence the compounds described in this invention may have synergistic activity since the molecules contain the DNA binder naphthalimide group.

### Objects of the invention

The main object of the present invention is to provide substituted 1*H-*benz[*de*]isoquinoline-1,3-diones of formula 3.

Another object of the present invention is to provide a process for preparation of substituted 1*H*-benz[*de*]isoquinoline-1,3-diones of formula 3 useful as anticancer agents. Yet another object of the present invention is to provide novel groups of compounds of formula 3 having low toxicity.

A further object of this invention is to prepare chloro compounds as N-[2-(Chloroethyl)]- and N-[3-(Chloropropyl)] of substituted naphthalimides of formula 3 which may have other application in chemical industry.

Yet another object of the invention is to provide compositions containing as an active ingredient one or more of the compounds, having the general formula 3.

Still another object of the invention is to provide the composition, which did not adversely affect haematopoiesis and had not displayed hepatotoxicity or nephrotoxicity at its optimum doses.

One more object of the invention is to provide the composition, which is to be used for the treatment of the cancer wherein major mode of cell death is due to apoptosis.

### Detailed description

Accordingly the present invention provides novel substituted 1*H*-benz[*de*]-isoquinoline 1, 3-diones of general formula 3, 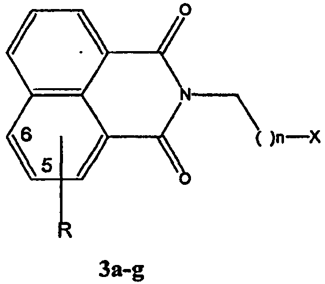 wherein R is selected from halogen, nitro; n=1 or 2; X=Cl, such that when n-1, R=6-Br or 6-NO₂ or 5-NO₂ and when n=2, R=6-Br or 6-cl or 6-NO₂ or 5-NO₂, or pharmaceutically acceptable salt thereof.

In an embodiment of the invention, the structural formula of substituted 1*H-*benz[*de*]-isoquinoline-1, 3-diones comprises (compounds 3a-c); 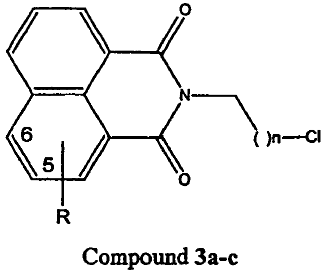 wherein n = 1; R = 6-Br, or 6-NO₂ or 5-NO₂

In another embodiment of the invention, the structural formula of substituted 1*H-*benz[*de*]-isoquinoline-1, 3-diones comprises (compounds **3d-g);** 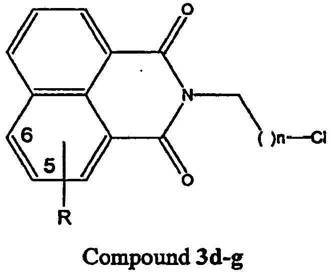 wherein n = 2; R = 6-Br or 6-Cl or 6- NO₂ or 5-NO₂

In yet another embodiment of the invention, the representative compounds of the general formula 3 comprises
(a) 6-Bromo-2-[2-(Chloroethyl)]-1H-benz[de]isoquinoline-1,3-dione
(b) 6-Nitro-2-[2-(Chloroethyl)]-1H-benz[de]isoquinoline-1,3-dione
(c) 5-Nitro-2-[2-(Chloroethyl)]-1H-benz[de]isoquinoline-1,3-dione
(d) 6-Bromo-2-[3-(Chloropropyl)]-1H-benz[de]isoquinoline-1,3-dione
(e) 6-Chloro-2-[3-(Chloropropyl)]-1H-benz[de]isoquinoline-1,3-dione
(f) 6-Nitro-2-[3-(Chloropropyl)]-1H-benz[de]isoquinoline-1,3-dione
(g) 5-Nitro-2-[3-(Chloropropyl)]-1H-benz[de]isoquinoline-1,3-dione,

In a further embodiment of the invention, novel substituted 1*H-*benz[*de*]-isoquinoline-1, 3-diones of general formula 3, and salts, thereof, 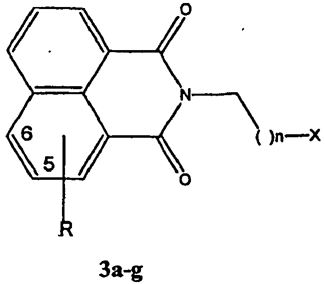 wherein R is selected from halogen, nitro; n=1 or 2; X=Cl, such that when n=1, R=6-Br or 6NO₂ or 5-NO₂ and when n=2, R=6-Br or 6-Cl or 6-NO₂ or 5-NO₂, are useful for the treatment of cancer.

Accordingly, the present invention provides a process for preparation of novel substituted 1*H*-benz[*de*]-isoquinoline-1,3-diones of general formula 3 and pharmaceutically acceptable salts thereof, 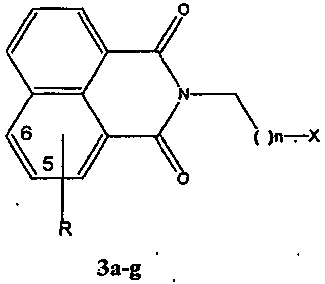 wherein R is selected from halogen and nitro; n =1 or 2; X = Cl, such that when n=1, R=6-Br or 6NO₂ or 5-NO₂ and when n=2, R=6-Br or 6-Cl or 6-NO₂ or 5-NO₂,
wherein the steps comprise; reacting substituted naphthalimide hydroxy derivatives of general formula 2a-g 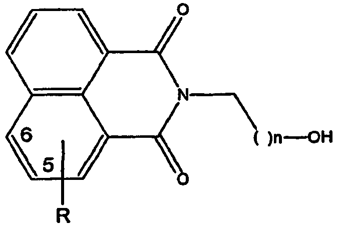 wherein R is 6-Br or 6-Cl or 6-NO₂ or 5-NO₂ and n=1 or 2, such that when n=1, R=6-Br or 6NO₂ or 5-NO₂ and when n=2, R=6-Br or 6-Cl or 6-NO₂ or 5-NO₂, with POCl₃ under inert and dry atmosphere at a temperature ranging between 80-90°C for a period ranging between 1 to 2 hr, evaporating excess POCl₃ to obtain the resultant and followed by purification by standard methods to obtain the desired compound.

In an embodiment of the invention, the reaction may be carried out under inert atmosphere using an inert gas preferably nitrogen.

The hydroxyl derivative may be selected from suitably substituted naphthalimides.

In yet another embodiment of the invention, the ratio of POCl₃ to the compound may be in the range of 2 to 3 molar times.

In a further embodiment of the invention, purification of the compound may be carried out by chromatographic method.

In an embodiment of the invention, the reaction may be carried out at a temperature ranging between 80 to 90°C.

A pharmaceutical composition, preferably for use in the treatment of cancer, comprises an effective amount of a compound of general formula 3, or salts thereof, wherein R is selected from halogen, nitro; n = 1 or 2; X = Cl, such that when n=1, R=6-Br or 6NO₂ or 5-NO₂ and when n=2, R=6-Br or 6-Cl or 6-NO₂ or 5-NO₂, optionally along with the pharmaceutical carrier, diluents, additives and lubricant, accordingly to one embodiment of the present invention.

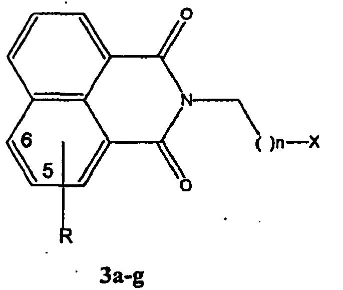

According to one embodiment of the invention, the pharmaceutical composition comprises an effective amount of a compound of general formula 3b and 3f, as defined in the claims.

Preferably, the effective dose of the composition is ranging between 30 to 80 mg /Kg body weight.

In another embodiment of the invention, the carrier used is selected from the group consisting of cellulosic suspending agents, polymeric suspending agents, xanthan gum, silicone dioxide and mixtures thereof.

In yet another embodiment of the invention, the diluent used is selected from the group consisting of lactose, dicalcium phosphate, mannitol and corn starch.

In a further embodiment of the invention, the additive used is selected from the group consisting of sodium citrate, calcium carbonate, dicalcium phosphate together with various additional substances such as starch, gelatin, etc.

In an embodiment of the invention, the lubricant used is selected from the group consisting of magnesium stearate, calcium stearate, glycerol monostearate, hydrogenated caster oil / hydrogenated vegetable oil, mineral oil, polyethylene glycol, sodium lauryl sulphate, etc.

In an embodiment of the invention, the subject is any animal including human suffering from cancer and/or related diseases.

In yet another embodiment of the invention, the composition is used at doses ranging between 30 to 80 mg/kg.

In an embodiment of the invention, the composition is administered by oral, intravenous, intraperitoneal, intramuscular, subcutaneous, intrauterine routes.

In a further embodiment of the invention, the composition may be used in the form of tablet, injectable syrup, capsule.

Preferably, the composition is effective for the tumor inhibition around 90%.

The tumor cell line used may be selected from the group consisting of Colon: 502713, HT-29, SW-620, HCT-15, COLO-205; Liver: Hep-2, Prostate DU-145, PC-3; Breast: MCF-7; Neuroblastoma: IMR-32, SK-N-SH; Ovary: OVCAR-5, SKOV-3; Lung: A549; Leukemia: HL-60 & Lymphoma: U-937. In an embodiment of the invention wherein the compound exhibited more than 50% growth inhibition at 10⁻⁵M against Colon: 502713, HT-29, SW-620, HCT-15, COLO-205; Liver: Hep-2, Prostate DU-145, PC-3; Breast: MCF-7; Neuroblastoma: IMR-32, SK-N-SH; Ovary: OVCAR-5, (Table 4).

Preferably, the in vitro cytotoxicity against cancer cell lines is more than 50% at 1x10⁻⁵M concentration, comparable to or higher than the standard drugs (table 4).

Preferably, *in vitro* cytotoxicity [IC₅₀ (µM)] values of 3b and 3f against HL-60 human leukemia cell line are 0.8µM and 0.30 µM which are lower than the standard drugs and hence more cytotoxic than the standards (table 5).

Preferably, the in *vitro* cytotoxicity [IC₅₀ (µM)] values of **3b** and **3f** against U-937 lymphoma line are 0.66µM and 1.95µM which are lower than the standard drugs and hence more cytotoxic than the standards (table 5).

Preferably, the compound 3f inhibits the DNA and RNA synthesis in vitro in S-180 tumor cells comparable to standard drug Mitonafide. Preferably, major mode of cell death is due to apoptosis in MOLT-4 cells.

In MOLT-4 the percentage of early apoptotic cells (Annexin V⁺/PI⁻) on treatment with compound 3f were 6.38%, and 13.63% at 3 & 5 µg/ml respectively.

In MOLT-4 the percentage of late apoptotic cells (Annexin V⁺/PI⁺) on treatment with compound 3f were 1.05%, and 4.82% at 3 & 5 µg/ml respectively.

In MOLT-4 the percentage of total apoptotic cells on treatment with compound 3f were 7.43%, and 18.44% at 3 & 5 µg/ml respectively which is higher than the standard compound camptothecin (8.83% at 5µM) whereas that of control is 1.35% only.

Preferably, the programmed cell death is measured using MOLT-4 wherein compound **3f** induces caspase-3 at 5 µg/ml at 12 hrs of incubation and caspase-6 at 5 µg/ml at 12 and 24 hrs.

DNA cell cycle analysis of MOLT-4 cells exposed to **Compound 3f** up to 24 hr treatment at concentration of 1.5 µg/ml to 5 µg/ml exhibited increase in sub-G₁ fraction (apoptotic) from 4.52 to 5.86 %, which may comprise both apoptotic, and debris fraction.

Also preferably, major mode of cell death is due to apoptosis also in HL-60 cells.

In HL-60 the percentage of early apoptotic cells (Annexin V⁺/PI⁻) on treatment with compound **3b** were 4.5% and 15.3% at 5 & 10µM respectively.

In HL-60 the percentage of late apoptotic cells (Annexin V⁺/PI⁺) on treatment with compound **3b** were 5.2% and 33.5% at 5 & 10µM respectively.

In HL-60 the percentage of total apoptotic cells on treatment with compound 3b were 48.8% at 10µM, which is much higher than the standard compound camptothecin (15.8% at 10µM).

In HL-60 the percentage of early apoptotic cells (Annexin V⁺/PI⁻) on treatment with compound **3f** were 16.4% and 11.5% at 5 & 10µM respectively.

In HL-60 the percentage of late apoptotic cells (Annexin V⁺/PI⁺) on treatment with compound **3f** were 31.5% and 81.0% at 5 & 10µM respectively.

In HL-60 the percentage of total apoptotic cells on treatment with compound 3f was 92.5% at 10µM, which is much higher than the standard compound camptothecin (15.8% at 10µM).

Preferably, the representative **compound 3f** at its optimum doses did not adversely affect haematopoiesis and had not displayed hepatotoxicity or nephrotoxicity in normal and in S-180 tumor bearing mice. Preferably, composition is active against tumor in mice and inhibits the S-180 tumor growth up to 80%.

Preferably, **compound 3f** possesses comparable anticancer activity compared to cyclophosphamide in the mouse S-180 tumor system when they are administered by intraperitoneal route (i.p.).

Preferably, the **compound 3f** exhibited greater inhibitory effect on DNA synthesis compared to RNA synthesis in the S-180 tumor system.

The value of T/C % (treated/control) was 138 in the mouse S-180 tumor system for the **compound 3f** at a dose of 60 mg/kg body weight.

The value of T/C % (treated/control) was 189 in the mouse EAC tumor system for the **compound 3f** at a dose of 60 mg/kg body weight.

In an embodiment of the invention the salt of the compounds of general formula 3 comprised in the composition may be any pharmaceutically acceptable salt such as hydrochloride, hydrobromide, or a salt of acetic acid, mesylic acid.

The present invention relates to novel substituted 1*H-*benz[*de*]isoquinoline-1,3-diones of general formula 3 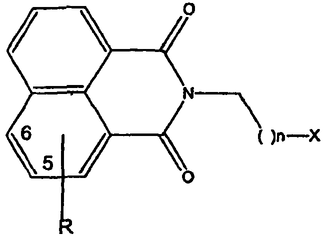 wherein n = 1-2; X = Cl; R = 6-Br, 6-Cl, 6-NO₂, 5-NO₂, as defined in the claims. N-[2-(Chloroethyl)]- **(Compounds 3a-c)** and N-[3-(Chloropropyl)]-naphthalimides **(Compounds 3d-g)** were synthesized as potential antitumor agents based on the structure of the experimental compound Mitonafide. The compounds were obtained from the corresponding known hydroxyl derivatives. *In vitro* screening of one of the most potent **Compound 3f** in sixteen human tumor cell lines namely Colon: 502713, HT-29, SW-620, HCT-15, COLO-205; Liver: Hep-2, Prostate DU-145, PC-3; Breast: MCF-7; Neuroblastoma: IMR-32, SK-N-SH; Ovary: OVCAR-5, SKOV-3; Lung: A549; Leukemia: HL-60 and Lymphoma: U-937, revealed significant cytotoxicity in fourteen cell lines except in two cell lines namely Ovarian SKOV-3 and Lung A549 at 10µM concentration or lower (1µM). *In vivo* anti-tumoral potency of the new **Compound 3f** was assessed by measuring the increase in median survival time of drug treated (T) over untreated control (C) mice in the murine ascites tumor Sarcoma-180 (S-180) at a dose of 60 mg/kg and it revealed marginal but statistically significant tumor growth inhibition (% T/C 138). Similar study with **Compound 3f** at the same dose in Ehrlich ascites carcinoma (EAC) revealed its significantly high activity (% T/C 189) in this tumor. Its toxicity was assessed *in vivo* in normal and S-180 bearing mice by measuring drug-induced changes in hematological parameters, femoral bone marrow and spleenic cellularities as well as hepatotoxicity and nephrotoxicity sequentially on days 9, 14 and 19 following drug treatment at the optimum dose of 60 mg/kg from day 1 to 7. Results indicate that it did not adversely affect hematopoiesis. Other parameters were within normal limit. **Compound 3f** comparable to standard (Mitonafide) inhibited the synthesis of DNA and RNA in S-180 tumor cells. It had exhibited slightly lower inhibitory effect on RNA synthesis than Mitonafide.

Flow cytometric analyses were carried out to differentiate between the inductions of apoptosis and/or necrosis in MOLT-4 cells by **Compound 3f.** Earlier event of apoptosis that was measured using Annexin V binding assay showed that early apoptotic cells (Annexin V⁺/PI⁻) on treatment with **Compound 3f** (3, 5 µg/ml) were 6.38 % & 13.62 % respectively. The late apoptotic cell population (Annexin V⁺/PI⁺) for **Compound 3f** (3, 5µg/ml) were 1.05% and 4.82% respectively. Hence the major mode of cell death appears to be apoptosis that was further confirmed by other parameters.

To further confirm programmed MOLT-4 cell death measured caspase-3/6 activity indicates that **Compound 3f** induces caspase-3 at 5 µg/ml at 12 hrs of incubation & caspase-6 at 5 µg/ml at 12 and 24 hrs.

DNA cell cycle analysis of MOLT-4 cells exposed to **Compound 3f** indicated that **Compound 3f** up to 24 hr treatment at concentration of 1.5 µg/ml to 5 µg/ml exhibited increase in sub-G₁ fraction (apoptotic) from 4.52 to 5.86 %, which may comprise both apoptotic, and debris fraction.

Morphological and ultra structural studies reveal that **Compound 3f** induces apoptosis in MOLT-4 cells. The apoptotic changes such as condensation of nucleus, marginalization of chromatin material and fragmentation of nucleus are clearly visible. At 36 hrs of 10µM of **Compound 3f** treatment, almost all the cells are seen to undergo apoptosis.

In our study we have discovered that substituted naphthalimide derivatives having N-(2-chloroethyl) and N-(3-chloropropyl) best represented by **compound 3f** possess interesting anti-tumoral activity in a number of experimental tumors. Further the representative **compound** 3f at its optimum doses did not adversely affect haematopoiesis and had not displayed hepatotoxicity or nephrotoxicity.

One new compound of the same type N-[3-(Chloropropyl)]naphthalimide **(compound 3f)** has been shown to possess insignificant bone marrow and spleenic toxicities.

It is also generally known that after some time tumors tend to develop resistance to the anticancer therapy employed, thus rendering the therapy without curative effect. Thus, the need exists for new and improved chemotherapeutic agents of this type, either or both with increased anti-tumor activity and decreased toxicity.

It has been found that the novel N-[2-(Chloroethyl)] and N-[3-(Chloropropyl)]-naphthalimides of the present invention possess improved chemotherapeutic properties in comparison with several known and clinically used anti-tumor agents and an experimental compound namely Mitonafide. The compounds of the invention show improved cytotoxic activity and apoptotic effect against various tumor cell lines. The main utility of this invention is to prepare hitherto unknown compounds, which may be useful in the treatment of human cancers.

In recent years ongoing research is focused towards the development of chemotherapeutic agents having properties other than the classical alkylating agents like apoptosis etc with a view to their plausible application in human cancers. Several structural patterns have been explored for this purpose. However, literature survey reveals that there are no reports, to our knowledge, in which the substituted naphthalimides have been used as such apoptotic agents. The naphthalimide ring was particularly chosen since it has been found that some substituted naphthalimides containing N-(2-dimethylaminoethyl) chain best represented by amonafide and mitonafide possessing DNA binding properties, have exhibited substantial cytotoxicities in various animal and human tumors and have undergone clinical trials. Hence the synthesized compounds described in this invention may have synergistic activity since the molecules contain the DNA binder naphthalimide group. 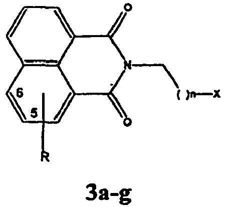 1. Compound having the formula: C₁₄H₉ClNO₂-R **(Compounds 3a-c,** where n = 1; X = Cl; R= 6-Br, 6-NO₂, 5-NO₂ **all are unknown)** and C₁₅H₁₁ClNO₂-R (Compounds **3d-g** where n = 2; X = Cl; R = 6-Br, 6-Cl, 6- NO₂, 5-NO₂ **all are** unknown).

### Nomenclature:

(a) 6-Bromo-2-[2-(Chloroethyl)]-1H-benz[de]isoquinoline-1,3-dione
(b) 6-Nitro-2-[2-(Chloroethyl)]-1H-benz[de]isoquinoline-1,3-dione
(c) 5-Nitro-2-[2-(Chloroethyl)]-1H-benz[de]isoquinoline-1,3-dione
(d) 6-Bromo-2-[3-(Chloropropyl)]-1H-benz[de]isoquinoline-1,3-dione
(e) 6-Chloro-2-[3-(Chloropropyl)]-1H-benz[de]isoquinoline-1,3-dione
(f) 6-Nitro-2-[3-(Chloropropyl)]-1H-benz[de]isoquinoline-1,3-dione
(g) 5-Nitro-2-[3-(Chloropropyl)]-1H-benz[de]isoquinoline-1,3-dione

Compound studied in detail from the group is 3f 6-Nitro-2-[3-(chloropropyl)]-1H-benz[de]isoquinoline-1,3-dione [n = 2; X = Cl; R = 6-NO₂]

### Chemical synthesis

### General procedure for the preparation of 2-[2-(Chloroethyl)] and 2-[3-(Chloropropyl)]-1H-benz[de]isoquinoline-1,3 dione (3a-g).

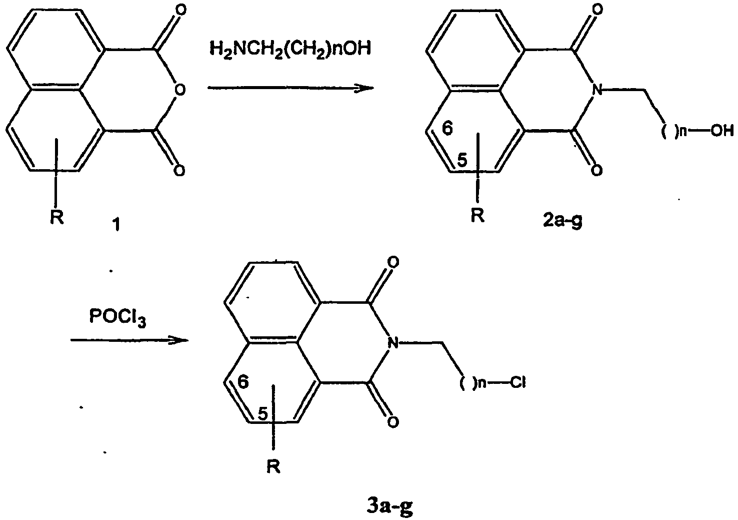

The starting known hydroxy derivatives 2a-g has been prepared by the known method disclosed in the literature.

Compound 2a [Grayshan, P.H.; Kadhim, A.M.; Peters, A.T. (1974). Heterocyclic derivatives of naphthalene-1,8-dicarboxylic anhydride. III. Benzo[k,l]thioxanthene-3,4-dicarboximides. J. Heterocycl Chem. 11, 33-8].

Compound 2b [Okazaki, M.; Suhara, Y.; Uemura, S.; Fujiyama, M.; Oda, K.; Tanaka, T.; Taniguchi, S.; Watanabe, Y. (1956). Derivatives of 4-aminonaphthalimide. IV. Synthesis of alkyl- and arylimides of 4-nitronaphthalic acid. Yuki Gosei Kagaku Kyokaishi 14, 455-61. Wardman, P.; Clarke, E.D.; Hodgkiss, R.J.; Middleton, RW.; Parrick, J.; Stratford, M. R L. (1984). Nitroaryl compounds as potential fluorescent probes for hypoxia. I. Chemical criteria and constraints. International J. Radiation Oncol. Biol. Phy. 10,1347-51. Middleton, RW.; Parrick, J.; Clarke, E.D.; Wardman, P. (1986). Synthesis and fluorescence of N-substituted-1,8-naphthalimides. J. Heterocycl. Chem. 23, 849-55].

Compound 2c [Brana, M.F.; Sanz, A.M.; Castellano, J.M.; Roldan, C.M.; Roldan, C. (1981). Synthesis and cytostatic activity of benz[de]isoquinoline-1,3-diones. Structure-activity relationships. Eur. J. Med. Chem. 16, 207-12. Hodgkiss, R J.; Middleton, R. W.; Stratford, M. R. L.; Del Buono, R. (1987). Toxicity of 3-nitronaphthalimides to V79 379A Chinese hamster cells. Biochem. Pharmacol. 36, 1483-7].

Compound 2d [Grayshan, P.H.; Kadhim, A.M.; Peters, A.T. (1974). Heterocyclic derivatives of naphthalene-1,8-dicarboxylic anhydride. III. Benzo[k,l]thioxanthene-3,4-dicarboximides. J. Heterocycl Chem. 11, 33-8].

Compound 2e [Kadhim, A.M.; Peters, A.T. (1974). New intermediates and dyes for synthetic polymer fibers. 4-(4-Methoxyanilino)-3-nitro-1, 8-naphtalimides. J. Soc. Dyers Colourists 90, 153-7. Minakova, R A.; Bedrik, A. I; Shershukov, V. M.; Surov, Yu. N.; Pivnenko, N. S. (1999). Synthesis of hydroxyl containing N-imides of 4-substituted naphthalic acid. Chemistry of Heterocyclic Compounds. Khimiya Geterotsiklicheskikh Soedinenii 35, 621-624].

Compound 2f [Grayshan, P.H.; Kadhim, A.M.; Peters, A.T. (1974). Heterocyclic derivatives of naphthalene-1,8-dicarboxylic anhydride. III. Benzo[k,l]thioxanthene-3,4-dicarboximides. J. Heterocycl Chem. 11, 33-8. Minakova, R. A.; Bedrik, A. I.; Shershukov, V. M.; Surov, Yu. N.; Pivnenko, N. S. (1999). Synthesis of hydroxyl containing N-imides of 4-substituted naphthalic acid. Chemistry of Heterocyclic Compounds. Khimiya Geterotsiklicheskikh Soedinenii 35, 621-624].

Compound 2g [Hirota, N; Taniguchi, T; Oda, T. (2001). Electrophotographic photoreceptor using charge-transporting unsaturated aniline-like compound and/or indoline-like compound associated with imide. *Jpn. Kokai Tokkyo Koho* 43 pp Patent No.JP 2001117247].

Substituted naphthalic anhydrides [Compound 1] were subjected to react with 2-aminoethanol or 3-aminopropanol to furnish the corresponding known hydroxy derivatives 2a-g which in turn (1 mmol) was mixed with POCl₃ (2 ml) under protection from atmospheric moisture in nitrogen atmosphere and the whole was heated at 80-90°C in an oil bath for 1.5 h. Excess POCl₃ was driven off by blowing air and the resulting residue was worked up as usual by extracting with chloroform followed by washing with brine and drying (Na₂SO₄). Solvent was concentrated to furnish a waxy solid. This was purified by column chromatography over silica gel prepared in pet. ether. Elution with pet. ether : chloroform (1:1, v/v) furnished the desired products as amorphous products (Table 1).

In general these compounds are obtained in good yield. It was found that the compounds are stable and can be stored under moisture-free condition in amber-colored screw-cap glass vessels in the dessicator for indefinite period. The ¹HNMR spectral data of compounds **3a-g** is given in Table 2.

### Characterization of compounds:

Compounds **3a-g** were characterized by PMR spectra measured in a Brucker 300 MHz spectrometer with the solvents as indicated and chemical shifts were expressed in δ units (ppm) using tetramethyl silane as internal standard. IR and UV spectra were recorded in Perkin-Elmer FT-IR model no RX-1 and Shimadzu model no. UV-160A respectively. TLC analysis was carried out with glass plates coated with Silica gel G (Solvent system CHCl₃ - Pet ether, v/v 50: 50). Purity was checked with Waters HPLC system at ambient temperature (M-501 / M-510 solvent delivery pump, M-481 UV-Vis variable - wavelength detector set at 255nm / 696 Photodiode Array detector, U6K injector, novapak C₁₈ steel column, isocratic mobile phase methanol-water in varying proportions at a flow rate of 0.5 ml/min. Data analysis were done in M-745B data module with recorder / Millennium 32 Chromatography Manager) (Table 3). Melting points were determined on a Thomas-Hoover Unimelt capillary melting point apparatus and were uncorrected.

### Structure of the compounds

| | | | | | |
|---|---|---|---|---|---|
| 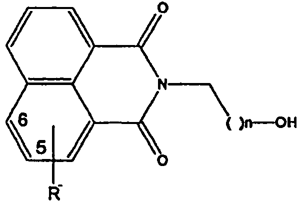 | | | 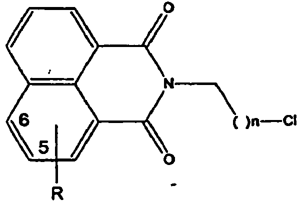 | | |

| **Compound 2** | | | **Compound 3** | | |
|---|---|---|---|---|---|
| n = 1, R = | 6-Br | **2a** | n = 1, R = | 6-Br | **3a** |
| | 6-NO₂ | **2b** | | 6-NO₂ | **3b** |
| | 5-NO₂ | **2c** | | 5-NO₂ | **3c** |
| n = 2, R = | 6-Br | **2d** | n = 2, R = | 6-Br | **3d** |
| | 6-Cl | **2e** | | 6-Cl | **3e** |
| | 6-NO₂ | **2f** | | 6-NO₂ | **3f** |
| | 5-NO₂ | **2g** | | 5-NO₂ | **3g** |

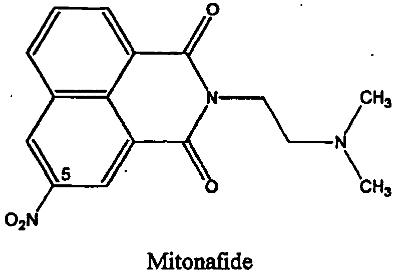

Following examples are given by way of illustration.

### Example 1

### Preparation of 6-Bromo-2-[2-(Chloroethyl)]-1H-benz[de]isoquinolino-1,3-dione (Compound 3a).

4-Bromonaphthalic anhydride (276 mg, 1 mmol) was refluxed with 2-aminoethanol (0.1ml, 1.5 mmol) in 1.5ml absolute alcohol for 2 hours. A yellow precipitate of the corresponding N-[2-(Hydroxyethyl)]naphthalimide compound 2a was obtained (224 mg). It was filtered, dried thoroughly over phosphorous pentoxide in vacuum. Crystallization of the crude compound from ethanol furnished pure compound (192 mg, 60%). 1 mmol (320 mg) of the hydroxy compound was mixed with 2 ml of POCl₃ and heated at 85°C in an oil bath under nitrogen for 1.5 h. Excess POCl₃ was driven off by blowing air and the resulting residue was worked up as usual by extracting with chloroform followed by washing with brine and drying over anhydrous Na₂SO₄. Solvent was concentrated to furnish a waxy solid (228 mg). This was purified by column chromatography over silica gel (5 gm) prepared in pet. ether. Elution with pet. ether : chloroform (1:1, v/v) furnished the desired product (203 mg, 60%) as amorphous product (Table 1).

### Example 2

### Preparation of 6-Nitro-2-[2-(Chloroethyl)]-1H-benz[de]isoquinoline-1,3-dione (Compound 3b).

4-Nitronaphthalic anhydride (242 mg, 1 mmol) was refluxed with 2-aminoethanol (0.1ml, 1.5 mmol) in 1.5ml absolute alcohol for 2 hours. A brown precipitate of the corresponding N-[2-(Hydroxyethyl)]naphthalimide compound 2b was obtained (254 mg). It was filtered, dried thoroughly over phosphorous pentoxide in vacuum. Crystallization of the crude compound from ethanol furnished pure compound (228 mg, 80%). 1 mmol (286 mg) of the hydroxy compound was mixed with 2 ml of POCl₃ and heated at 85°C in an oil bath under nitrogen for 1.5 h. Excess POCl₃ was driven off by blowing air and the resulting residue was worked up as usual by extracting with chloroform followed by washing with brine and drying over anhydrous Na₂SO₄. Solvent was concentrated to furnish a waxy solid (265 mg). This was purified by column chromatography over silica gel (5 gm) prepared in pet. ether. Elution with pet. ether : chloroform (1:1, v/v) furnished the desired product (210 mg, 69%) as amorphous product (Table 1).

### Example 3

### Preparation of 5-Nitro-2-[2-(Chloroethyl)]-1H-benz[de]isoquinoline-1,3-dione (Compound 3c).

3-Nitronaphthalic anhydride (242 mg, 1 mmol) was refluxed with 2-aminoethanol (0.1ml, 1.5 mmol) in 1.5ml absolute alcohol for 2 hours. A yellow/brown precipitate of the corresponding N-[2-(Hydroxyethyl)]naphthalimide compound 2c was obtained (260 mg). It was filtered, dried thoroughly over phosphorous pentoxide in vacuum. Crystallization of the crude compound from ethanol furnished pure compound (237.5 mg, 83%). 1 mmol (286 mg) of the hydroxy compound was mixed with 2 ml of POCl₃ and heated at 85°C in an oil bath under nitrogen for 1.5 h. Excess POCl₃ was driven off by blowing air and the resulting residue was worked up as usual by extracting with chloroform followed by washing with brine and drying over anhydrous Na₂SO₄. Solvent was concentrated to furnish a waxy solid (292 mg). This was purified by column chromatography over silica gel (5 gm) prepared in pet. ether. Elution with pet. ether : chloroform (1:1, v/v) furnished the desired product (253 mg, 83%) as amorphous product (Table 1).

### Example 4

### Preparation of 6-Bromo-2-[3-(aminopropyl)]-1H-benz[de]isoquinoline-1,3-dione (Compound 3d).

4-Bromonaphthalic anhydride (276 mg, 1 mmol) was refluxed with 3-aminopropanol (0.2ml), 2.6 mmol) in 1.5ml absolute alcohol for 2 hours. A yellow precipitate of the corresponding N-[3-(Hydroxypropyl)]naphthalimide compound 2d was obtained (290 mg). It was filtered, dried thoroughly over phosphorous pentoxide in vacuum. Crystallization of the crude compound from ethanol furnished pure compound (251 mg, 75%). 1 mmol (334 mg) of the hydroxy compound was mixed with 2 ml of POCl₃ and heated at 85°C in an oil bath under nitrogen for 1.5 h. Excess POCl₃ was driven off by blowing air and the resulting residue was worked up as usual by extracting with chloroform followed by washing with brine and drying over anhydrous Na₂SO₄. Solvent was concentrated to furnish a waxy solid (300 mg). This was purified by column chromatography over silica gel (5 gm) prepared in pet. ether. Elution with pet. ether : chloroform (1:1, v/v) furnished the desired product (264 mg, 75%) as amorphous product (Table 1).

### Example 5

### Preparation of 6-Chloro-2-[3-(Chloropropyl)]-1H-benz[de]isoquinoline-1,3-dione (Compound 3e).

4-Chloronaphthalic anhydride (232 mg, 1 mmol) was refluxed with 3-aminopropanol (0.2ml, 2.6 mmol) in 1.5ml absolute alcohol for 2 hours. A yellow precipitate of the corresponding N-[3-(Hydroxypropyl)]naphthalimide compound 2e was obtained (275 mg). It was filtered, dried thoroughly over phosphorous pentoxide in vacuum. Crystallization of the crude compound from ethanol furnished pure compound (258 mg, 89%). 1 mmol (290 mg) of the hydroxy compound was mixed with 2 ml of POCl₃ and heated at 85°C in an oil bath under nitrogen for 1.5 h. Excess POCl₃ was driven off by blowing air and the resulting residue was worked up as usual by extracting with chloroform followed by washing with brine and drying over anhy. Na₂SO_{4.} Solvent was concentrated to furnish a waxy solid (292 mg). This was purified by column chromatography over silica gel (5 gm) prepared in pet. ether. Elution with pet. ether: chloroform (1:1, v/v) furnished the desired product (274 mg, 89%) as amorphous product (Table 1).

### Example 6

### Preparation of 6-Nitro-2-[3-(Chloropropyl)]-1H-benz[de]isoquinoline-1,3-dione (Compound 3f).

4-Nitronaphthalic anhydride (242 mg, 1 mmol) was refluxed with 3-aminopropanol (0.2ml, 2.6 mmol) in 1.5ml absolute alcohol for 2 hours. A brown precipitate of the corresponding N-[3-(Hydroxypropyl)]naphthalimide compound 2f was obtained (290 mg). It was filtered, dried thoroughly over phosphorous pentoxide in vacuum. Crystallization of the crude compound from ethanol furnished pure compound (270 mg, 90%). 1 mmol (300 mg) of the hydroxy compound was mixed with 2 ml of POCl₃ and heated at 85°C in an oil bath under nitrogen for 1.5 h. Excess POCl₃ was driven off by blowing air and the resulting residue was worked up as usual by extracting with chloroform followed by washing with brine and drying over anhydrous Na₂SO₄. Solvent was concentrated to furnish a waxy solid (230 mg). This was purified by column chromatography over silica gel (5 gm) prepared in pet. ether. Elution with pet. ether : chloroform (1:1, v/v) furnished the desired product (197 mg, 62%) as amorphous product (Table 1).

### Example 7

### Preparation of 5-Nitro-2-[3-(Chloropropyl)]-1H-benz[de]isoquinoline-1,3-dione (Compound 3g).

3-Nitronaphthalic anhydride (242 mg, 1 mmol) was refluxed with 3-aminopropanol (0.2ml, 2.6 mmol) in 1.5ml absolute alcohol for 2 hours. A brown precipitate of the corresponding N-[3-(Hydroxypropyl)]naphthalimide compound 2g was obtained (282 mg). It was filtered, dried thoroughly over phosphorous pentoxide in vacuum. Crystallization of the crude compound from ethanol furnished pure compound (255 mg, 85%). 1 mmol (300 mg) of the hydroxy compound was mixed with 2 ml of POCl₃ and heated at 85°C in an oil bath under nitrogen for 1.5 h. Excess POCl₃ was driven off by blowing air and the resulting residue was worked up as usual by extracting with chloroform followed by washing with brine and drying over anhydrous Na₂SO₄. Solvent was concentrated to furnish a waxy solid (261 mg). This was purified by column chromatography over silica gel (5 gm) prepared in pet. ether. Elution with pet. ether : chloroform (1:1, v/v) furnished the desired product (204 mg, 64%) as amorphous product (Table 1).

### Example 8

### HPLC analysis

The retention times of the compounds with isocratic methanol-water as the flow solvent in the reverse-phase mode were shown where the most polar solute would be eluted first. The observed order of elution was as expected for the compounds. Thus compound 3c appears to be the most polar compound as it was least retained.

### Example 9

### 9a: In vitro screening in human tumor lines

Compounds 3a-g were screened by sulforhodamine-B (SRB) semi-automated assay in a battery of cell lines. Based on their initial activity, the most potent Compound 3f was further screened in fourteen human tumor cell lines namely Breast: MCF-7; Neuroblastoma: IMR-32, SK-N-SH; Colon: 502713, COLO-205, HCT-15, HT-29, SW-620, Liver: Hep-2; Lung: A549; Ovary: OVCAR-5, SKOV-3 and Prostate: DU-145, PC-3 according to the standard protocol [Monk. A., et. al. (1991); Feasibility high flux anticancer screen utilizing diverse panel of human tumor cell lines in culture. J Natl Cancer Inst. 83, 757-766]. Each compound was tested in triplicate sets of experiments at 10⁻⁴ to 10⁻⁶ M concentrations (Table 4).

### Preparation of test material:

A stock solution of 2 x 10⁻²M was prepared in DMSO. The stock solutions were serially diluted to obtain working test solutions with complete growth medium (RPMI-1640 medium with 2mM glutamine, 100µg/ml streptomycin, pH 7.4, sterilized by filtration and supplemented with 10% fetal bovine serum and 100 units/ml penicillin before use) containing 50 µg/ml of gentamycin to obtain working test solutions. The working test solutions were not filtered/ sterilized but microbial contamination was controlled by addition of gentamycin in complete growth medium used for dilution of stock solutions to prepare working test solutions.

### In vitro cytotoxicity:

***In vitro*** cytotoxicity against human cancer cell lines was determined² [Monk. A., et. al. (1991); Feasibility high flux anticancer screen utilizing diverse panel of human tumor cell lines in culture. J Natl Cancer Inst. 83, 757-766] using 96-well tissue culture plates. The cells were grown in tissue culture flasks in complete growth medium at 37°C in an atmosphere of 5% CO₂ and 95% relative humidity in a carbon dioxide incubator. The cell suspension of required cell density (1-2 lakhs/ml) depending on the mass doubling time of cell lines was prepared in complete growth medium for determination of cytotoxicity. The aliquots of 100 µl of cell suspension were added to each well on a 96-well tissue culture plate. The blank wells contained complete medium in place of cell suspension. Simultaneously, control experiments with positive controls containing known anticancer agents were carried out. Cells were incubated for 24 hr.

The test materials (100 µl in each well) were added after 24-hours to the wells containing cell suspension and blank wells. The cells were allowed to grow in presence of test material by further incubating the plates for 48 hours. At the end of incubation period the cell growth was stopped by gently layering trichloroacetic acid (50% TCA, 50µl/well) on top of the medium in all the wells. The plates were incubated at 4°C for 1 hr. The plates were washed five times with distilled water to remove TCA, growth medium, low molecular weight metabolites, serum proteins etc. and air-dried.

The cell growth was measured³ [Skehan, P, Storeng, R Scudiero, D, Monks, A, Mcmahon, J, Vistica, D, Warren, J.T., Bokesch, H., Kenney, S., Boyd, M.R. (1990). New colorimetric cytotoxicity assay for anti-cancer drug screening. J Natl Cancer Inst. 82: 1107-12] by staining with Sulphorhodamine B. Sulphorhodamine B (SRB, 0.4% in 1% acetic acid, 100 µl/well) was added to each well and plates were allowed to stand at room temperature for 30 minutes. The plates were washed with 1% acetic acid four times and then dried. Tris-HCl buffer (0.01 M, pH 10.5, 100 µl/well) was added to each well to solublise the dye. The plates were shaken gently for 10 minutes on a shaker and the optical density was recorded on ELISA reader at 540 nm. The cell growth was determined by subtracting mean OD value of respective blank from the mean OD value of experimental set. Percent growth in presence of test material was calculated considering the growth in absence of any test material as 100% and in turn percent growth inhibition in presence of test material was calculated. All the experiments were carried out in quadruplicate.

*In vitro* screening in fourteen human tumor cell lines namely Breast: MCF-7; Neuroblastoma: IMR-32, SK-N-SH; Colon: 502713, COLO-205, HCT-15, HT-29, SW-620, Liver: Hep-2; Lung: A549; Ovary: OVCAR-5, SKOV-3 and Prostate: DU-145, PC-3 revealed considerable (growth inhibition: >50% at 10⁻⁵ M) cytotoxic potential of **Compound 3f** against all the cell lines except for Ovarian: SKOV-3 and Lung: A549. Compound **3b** exhibited more than 50% growth inhibition at 10⁻⁵M against Neuroblastoma: IMR-32 and Colon: 502713, HCT-15, SW-620 (Table 4).

### 9b: In vitro screening in human tumor lines

The Compounds were further screened in HL-60 human leukemia and U-937 lymphoma lines using slightly different method. Drug stock solutions were prepared in DMSO [20mg of Compound / 1ml of HYBRIMAX grade, Sigma, USA] to obtain working stock solutions. These were serially 10-fold diluted with complete sterile growth medium RPMI-1640 with 2mM glutamine containing 10% fetal bovine serum and 1% antibiotics [100 U penicillin/ml and 100 µg streptomycin/ml] prior to use to obtain different drug concentrations. 4 clinical drugs as Cis-Platinum, BCNU, Hydroxyurea and 5-FU were included as standards. 100 µl of cell suspension from the stock 0.2 x 10⁵ HL-60 or 0.1 x 10⁵ U-937 were added to 96-well cell culture plates. 10 µl of drug solutions of different concentrations were added to respective wells followed by the addition of the medium (90 µl) in triplicate (total volume 200 µl/well). All vehicle controls contained the same concentration of DMSO. Plates were incubated for 96 h at 37°, 5% CO₂/95% air with humidity. After removal of 100 µl of media from each well, 10 µl of a 5 mg/ml solution of MTT in Dulbecco's PBS (GIBCO, BRL) was added to each well and the plate was incubated for 4 hr at 37°. Subsequently 100 µl of acid isopropanol solution (0.04 N HCl in isopropanol) was added to the wells and the formed formazan crystals were dissolved. The plate was read in a microplate reader at 540 nm. Curvefit software was used to calculate the IC₅₀ value (Table 5).

*In vitro* screening in the human tumor cell line Leukemia HL-60 and lymphoma U-937 lines, revealed significant cytotoxicity of the compounds 3b and 3f. Both of them are much more cytotoxic than the four standard drugs tested (Table 5).

### Example 10

### 10a: Analysis of apoptosis in MOLT-4 cells

### Annexin V FITC labeling of cells:

Events of apoptosis were analyzed using Annexin V-FITC Apoptosis Detection Kit (A 2214 Sigma Aldrich, USA). Human leukemia Cells (MOLT-4, 1x10⁶ cells/ml) in 6-well plate were treated with **Compound 3f** (3 and 5µg/ml), camptothecin (5µM) and incubated for 6 hrs. After incubation, the cells were centrifuged, rinsed with PBS twice and re-suspended in binding buffer (10 mM HEPES/NaOH, pH 7.5 containing 0.14M NaCl and 25 mM CaCl₂). An aliquot (500 µl) of suspension was stained with of Annexin V-FITC (5 µl) and Propidium Iodide (10 µl). The cells were stored for 10 minutes at room temperature in the dark and then analyzed by BD-LSR flow cytometer (Becton Dickinson, USA) at two wavelengths to measure fluorescence for of Annexin V & PI binding at 515 and 639 nm.

It was observed that early apoptotic cell population (Annexin V⁺/PI⁻) on treatment with **Compound 3f** were 638%, and 13.63% at 3 & 5 µg/ml respectively. The late apoptotic cell population (Annexin V⁺/PI⁺) for **Compound 3f** (3, 5µg/ml) were 1.05% and 4.82% respectively. Thus the percentage of total apoptotic cells on treatment with compound 3f were 7.43%, and 18.44% at 3 & 5 µg/ml respectively which is higher than the standard compound camptothecin (8.83% at 5µM) whereas that of control is 1.35% only.

That the major mode of cell death is apoptosis was also confirmed by other parameters.

### Caspase-3/6 activities:

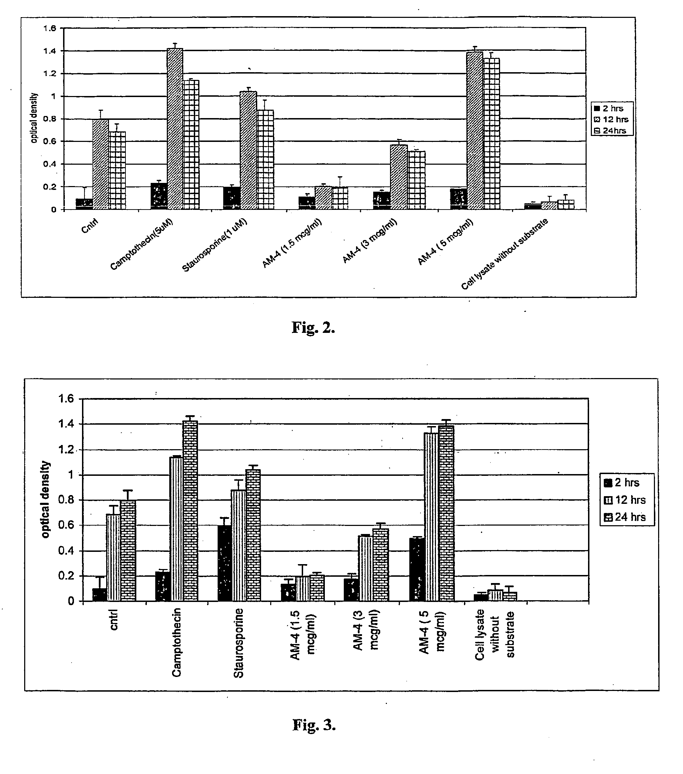

Caspase 3 activity was measured by using caspase-3 colorimetric Assay kit (R&D Systems USA). Briefly, MOLT-4 (∼2x10⁶cells) were treated with **Compound 3f,** camptothecin (5µM) and staurosporine (1µM) for 24h, and then cell centrifuged and pellet was lysed using 50µL cell lysis buffer. Cell lysates were mixed with 10µL of dithiothreitol (DTT) and 50µL reaction buffer (2X) containing 5 µL DEVD-pNA, a caspase-3 substrate, and incubated upto 24 hr at 37°C. Additional controls were included in this assay containing no cell lysate without substrate Enzyme-catalyzed release of pNA was monitored using a microplate reader at 405 nm (Fig. 2). Likewise, Caspase 6 activity was also measured using caspase-6 colorimetric Assay kit (R&D Systems USA). However, the enzyme and enzyme substrate (VEID-pNA) were different (Fig. 3). Treatment of the cells with Compound 3f induced caspase-3/6 activities. Caspase-3 activity of **Compound 3f** was maximum at 5 µg/ml at 12 hrs. which was more or less equivalent to camptothecin (5µM) for 12 hrs. (Fig 2). Caspase-6 activity of **Compound 3f** was maximum at 5 µg/ml for 24 hrs (Fig 3).

### Flow-cytometric-analysis of DNA cell cycle phase distribution of nuclear DNA

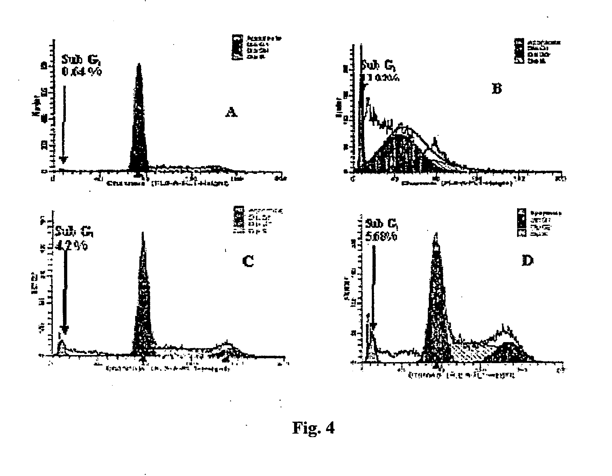

Effect of DNA content by cell cycle phase distribution was assessed using MOLT-4 cells. The cells (1 X 10⁶ were incubated with **Compound 3f** (3, 5 µg/ml), camptothecin (1µM). The incubation period was 3 hrs in case of camptothecin and 24 hrs. in rest of the treatments. The cells were then washed twice with ice-cold PBS, harvested, fixed with ice-cold PBS in 70% ethanol, and stored at -20 °C for 30 min. After fixation, the cells were incubated with RNase A (0.1 mg/ml) at 37°C for 30 min, stained with propidium iodide (50 µg/ml) for 30 min on ice in dark, and then measured for DNA content using BD-LSR Flow cytometer (Becton Dickinson, USA) equipped with electronic doublet, discrimination capability using blue (488nm) excitation from argon laser. Data were collected in list mode on 10,000 events for FL2-A vs. FL2-W and analyzed using Mod Fit 2.0 software (Fig 4). **Compound 3f** exhibited concentration dependent increase in sub-G₁ fraction in MOLT-4 cells which may comprise both apoptotic and debris fraction implying together the extent of cell death (Fig. 4C-D). The sub-G₁ fractions for control and camptothecin were 0.64 (Fig. 4A) and 11.92 % (Fig. 4B).

### 10b: Analysis of apoptosis in HL-60 cells

Induction of apoptosis in vitro by Compound **3b** and **3f** was further determined in HL-60 cells by flow cytometric assay using an Annexin V-FITC Apoptosis Detection Kit (BD Biosciences Pharmingen, San Diego, USA). Camptothecin was included in the study for comparison. Exponentially growing HL-60 cells in complete RPMI-1640 media (100 µl of 5×10⁶) were plated in six well plates. 10 µl of each Compound in DMSO (5 and 10 µM concentrations) was added to the wells followed by the addition of required media (total volume 1ml). Plates were incubated at 37°C for 24 h. The cells were centrifuged at room temperature at 1500 rpm for 5 min. The pellet was washed twice with cold PBS and was re-suspended in the binding buffer (1X, 100µl) provided in the Kit. The cells were stained with Annexin V-FITC (5 µl) and Propidium Iodide [PI] (5µl) and incubated for 15 min in the dark at 25°C. Next 400µl of binding buffer was added to each tube and analyzed on a FACScan flow cytometer (Becton Dickinson, Mountainview, CA) using Cell Quest software. Cells stained with both Annexin-V-FITC and PI, containing DMSO as vehicle was used as stained control. Cells that were annexin⁺ /Pr [Lower right quadrant (LR)] and annexin⁺/PI⁺ [Upper right quadrant (UR)] were considered as early apoptotic and late apoptotic respectively.

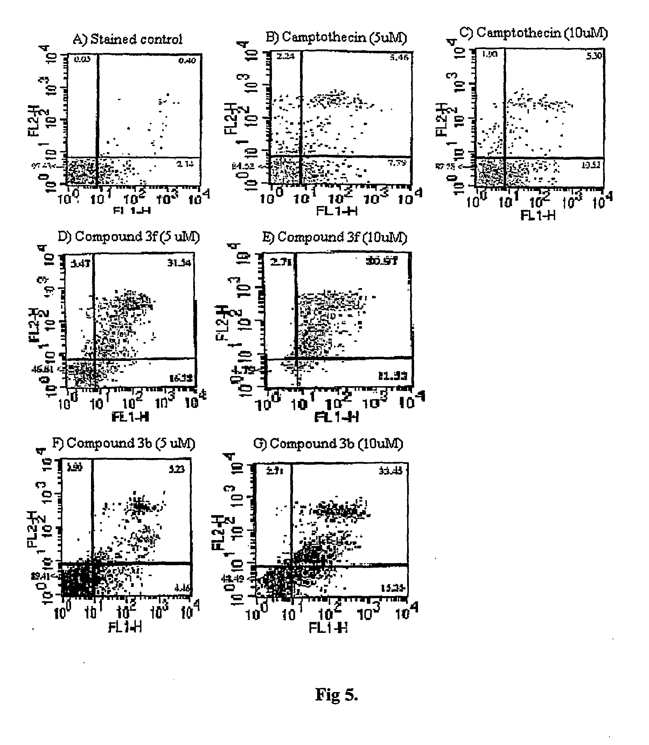

The results obtained for the Compounds have been compared with that of camptothecin, the well-known apoptosis inducer. Exposure of cells to Camptothecin (5 and 10 µM concentrations) resulted to 7.79% & 10.52% in LR, and 5.46% & 5.30% in UR respectively (Fig. 5B-C). Compound **3b** (5 and 10 µM concentrations) exhibited 4.5% & 15.3% in LR and 5.2% & 33.5% in UR (Fig. 5F-G). Compound **3f** (5 and 10 µM concentration) exhibited 16.4 & 11.5% in LR, and 31.5% & 81.0% in UR (Fig. 5D-E). Thus they induce apoptosis to a much greater extent than camptothecin at 10µM concentration.

### Example 11

### In vivo anticancer activity:

### Increase in the median survival time:

**Drug:** On the basis of the *in vitro* screening results, compound **3f** was selected for *in vivo* evaluation. Representative other compounds **3b** and **3g** were also studied.

**Mice:** Closed colony bred Swiss albino male mice of about 6-8 weeks of age weighing 24 ± 2 g were obtained from the Institute's vivarium, maintained and divided in two groups as control (untreated) and treated. At least 6 animals were used for a particular group in an experiment.

**Vehicle and route:** Physiological saline containing 2% Tween 80 was used for drug administration through intraperitoneal (i.p.) injection. The drug solutions were prepared daily just prior to the injection.

**Tumor:** S-180 and EAC cells obtained from National Centre for Cell Sciences, Pune (India), were used. Tumor cell suspensions in physiological saline were prepared to final concentrations of 5.0 x 10⁶ cells/ml as described⁴ [Samanta S, Pain A, Dutta S, Saxena AK, Shanmugavel M, Pandita RM, Qazi GN, Sanyal U. (2005). Antitumor activity of Nitronaphthal-NU, a novel mixed-function agent. J Exp Ther Oncol. 5:15-22]. Mice were inoculated with 5.0 x 10⁶ viable cells/ml at an injection volume of 0.2 ml on day 0 thus having 10⁶ cells/mouse for *in vivo* study.

**Increase in the median survival time: Compounds** in respective doses were administered to each mouse in treated groups as per Table 6. The control groups received an equal volume of vehicle (0.2 ml) on those days. The number of deaths were counted daily during the test. The testing was evaluated by calculating the median survival times (M.S.T.)⁵ [Geran RI, Greenberg NH, MacDonald MM, Schumacher AM, Abbott BJ. (1972). Protocols for screening chemical agents and natural products against animal tumors and other biological systems. Cancer Chemother Rep 3: Part 3, 1-103.] of drug-treated (T) and control (C) tumor-bearing animals and expressed as percent T/C value. A T/C percentage value > 125 was considered as significant Two standard clinical drugs as cyclophosphamide and 5-fluorouracil (5-FU) as well as the experimental compound Mitonafide were used as positive controls for comparison.

Single doses of compounds were administered to normal mouse to get the maximum tolerated dose. Groups of 8 mice were taken per dose. Following each dose of treatment, numbers of survivors were recorded on each day up to a period of 30 days from the date of injection. It was found that mouse could tolerate the single dose of 400 mg/kg per day of new compounds **3.** The higher doses cannot be applied since the compounds get precipitated in the vehicle.

Data for **Compound 3f** in S-180 revealed that the maximum T/C value of 138 was obtained at the dose of 60 mg/kg for QD₁₋₇ (Expt. no. 2, Table 6). Screening results were confirmed by repeating experiment with optimum dose. Thus **Compound 3f** has exhibited marginal but statistically significant but activity in S-180. Similar study with **Compound 3f** at the same dose and schedule in Ehrlich ascites carcinoma (EAC) revealed its significantly high activity (% T/C 189) in this tumor (Expt. no. 7, Table 6).

It was found that under similar treatment schedule and route (QD₁₋₇, i.p.), Mitonafide administered at the optimum dose of 0.5 mg/kg has displayed a T/C value of 187 in S-180. 5-FU has also demonstrated highly significant anticancer activity in these tumor systems having long-term survivors (T/C value in S-180 is 233).

It was found that the title **Compound 3f** has shown comparable anticancer activity compared to cyclophosphamide in these tumor systems when they are administered i.p. This may be due to the reason that cyclophosphamide differs from other compounds in that it requires a multistep activation process before functioning as an anti-tumor agent

### In vivo tumor growth inhibition study:

**Compound 3f** was selected for tumor growth inhibition study. 18 mice each were taken for treated and control groups and inoculated with 1 x 10⁶ S-180 cells. After sacrificing mice from the respective groups sequentially on days 9, 14 and 19 following drug treatment on day 1-7 at doses indicated, the ascites fluid from peritoneal cavities of animals were quantitatively transferred and the peritoneal cavities were washed with measured volume of physiological saline. The total number of tumor cells were counted and ascites fluid volume were measured. Averages ( x̅ ± S.E.M.) were made of these two parameters and the percentage inhibitions [(1 - T/C) ×100] were calculated for each dose.

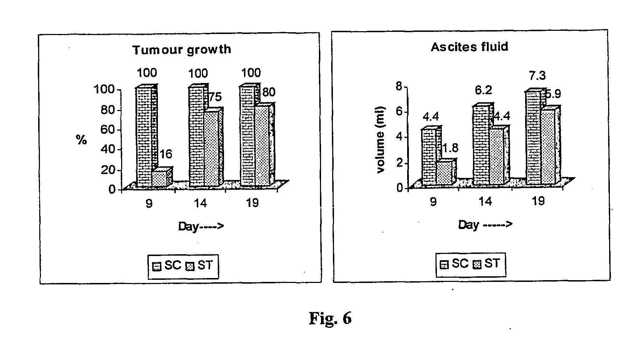

Results showed that initially there was a significant inhibition (84%) in S-180 tumor growth on day 9 after application of Compound 3f. Thereafter gradual growth of the tumor was noted and on 19^{th} day there was about 20% inhibition. Similar observation was noted for ascites fluid formation (Fig. 6).

### Example 12

### Microscopic studies:

**Cell culture and treatment:** Human T lymphoblast; acute lymphoblastic leukemia line MOLT-4 was procured from NCCS, Pune, India. The cells were cultured in RPMI-1640 medium supplemented with 10% FCS, 100 units penicillin and 100 µg streptomycin per ml. Cells were grown in CO₂ incubator (SHEL-LAB, USA) at 37° C, 90% humidity and 5% CO₂ gas environment and were treated during sub-confluent stage with **Compound 3f** dissolved in DMSO at the concentration of 1, 5 and 10µM while control cells received only the vehicle (DMSO < 0.5%).

**Light and Transmission Electron Microscopy:** MOLT-4 control, **Compound 3f** treated cells at different time intervals were washed in phosphate buffered saline, centrifuged at 1500 rpm for ten minutes and the pellets were divided into 1mm³ pieces and fixed immediately in 2.5% glutaraldehyde in 0.1M phosphate buffer (pH 7.2) for 2 hr at 4°C, post-fixed with 1% OsO₄ in the same buffer for 2 hr, dehydrated with acetone, cleared in propylene oxide and embedded in Epon-812⁶ [Hayat, M. A. (1981) Principles and Techniques of Electron Microscopy. Biological Applications Vol. 1. Edward Arnold Publishers, London, U.K.] Semithin (1µm) sections were cut, stained with toluidine blue and studied under Olympus Research Microscope (VANOX). The photography was done using Olympus Digital Camera (C4000).

Ultrathin sections of silver colour (60-90 nm) were cut on a LKB ultramicrotome (Ultrotome-IV, mounted on copper grids and stained with uranyl acetate and lead citrate. The sections were viewed and photographed in a JEOL-100CXII electron microscope at 60 kV.

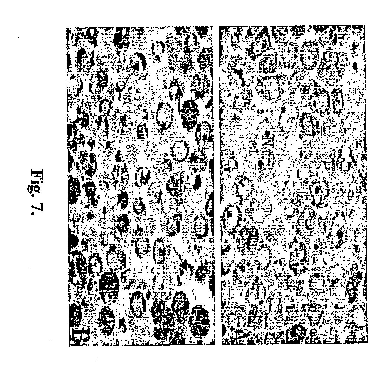

**Light Microscopy:** The morphology of the cells after treatment was observed in toluidine blue stained resin sections under light microscope as described⁷ [Stadelmann, C. and Lassmann, H. (2000). Detection of apoptosis in tissue sections. Cell Tissue Res. 301: 19-31] at different time intervals to study the mechanism of cell death. At 5µM, of **Compound 3f** treatment only a-few apoptotic cells were observed after 24 - 48 hr of treatment whereas no apoptotic cells were observed at 1µM. At 10µM **Compound 3f,** after 16 hrs of treatment, the apoptosis was observed which went on increasing and was seen in majority of cells after 36 hrs of treatment. **Compound 3f** treated MOLT-4 cells, the condensation of the nuclear material, with a marked accumulation of densely stained chromatin was seen. In majority of the treated cells the chromatin was seen typically at the edge of the nucleus. This was accompanied by cell shrinkage and cytoplasmic vacuoles. The disintegration of the nucleus into smaller fragments was also seen (Fig. 7B). These changes indicate that cells undergo apoptosis by treatment of **Compound 3f.** The control cells showed large sized nuclei having nucleoli and the cytoplasm was restricted to the periphery of the cells (Fig. 7A). However, no inflammation in nuclei and cytoplasm; plasma membrane breaks were visible on treatment of Compound 3f, which rules out the possibility of necrotic events. Similar observations have been reported for **HL-60** cells during apoptosis⁸ [Reno, F., Tontini, A., Burattini, S., Papa, E., Falcieri, E. and Tarzia, G. (1999). Mimosine induces apoptosis in the HL60 human tumor cell line. Apoptosis 4: 469-477].

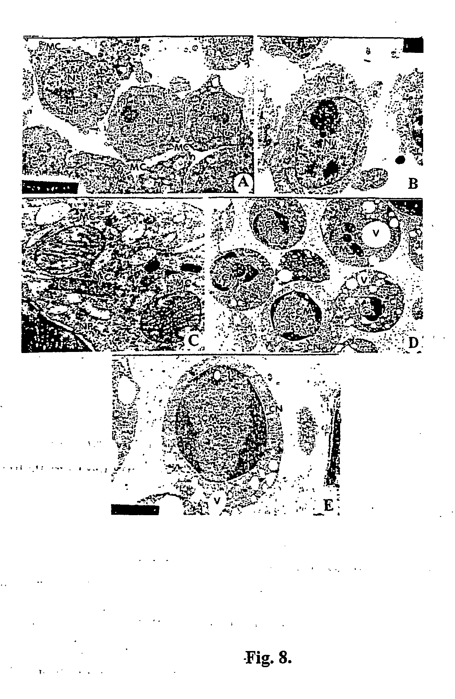

**Transmission Electron Microscopy:** We performed an electron microscopy study to analyze the morphological changes that **Compound 3f** caused in MOLT-4 cells. Ultra structural morphology of control cells (Fig. 8A-C) and apoptotic cells are shown in Fig. 8D-E Ultra structurally the MOLT-4 cells in control exhibited a high nucleocytoplasmic ratio. The nucleus has a finely dispersed chromatin. The nucleoli are identified in majority cells. The cytoplasm contains mitochondria, which show variation in size and shape, ribosomes and a few strands of rough endoplasmic reticulum (Fig. 8A-C). The nuclear pores were also seen in control cells (Fig. 8C). The control cells (Fig. 8C) showed two types of mitochondria one oval shaped and other elongated type.

After 36 hrs of 10µm **Compound 3f** treatment the majority of MOLT-4 cells were observed to undergo apoptosis (Fig. 8D-E). As shown in Fig. 8D-E, the condensation of the nucleus, chromatin marginlisation, typical of apoptosis in **Compound 3f** treated cells were clearly visible. The vacuolization was also seen in treated cells. Similar observations have been reported^{9a-b} [Gazzanelli, G., Luchetti, F., Burattini, S., Mannello, F., Falcieri, E. and Papa, S. Matrix metalloproteinases expression in HL-60 promyelocytic leukemia cells during apoptosis. Apoptosis. 5 (2000): 165-172; Ramirez, C. D. and Catchpoole, D. R. (2002) Etoposide-induced apoptosis in lympho-blastoid leukaemic MOLT-4 cells: Evidence that chromatin condensation, loss of phosphatidylserine asymmetry and apoptotic body formation can occur independently. Apoptosis. 7: 59-68] for HL-60 and MOLT-4 cells during apoptosis. Mitochondria play a key role in pathways to apoptosis. Mitochondrial morphology is important because changes in mitochondrial ultrastructure modulate mitochondrial function. The results of our study showed that mitochondrial cristae were damaged and rough endoplasmic reticulum was highly reduced in apoptotic cells.

Compound 3f has been shown to induce apoptosis in MOLT-4 cells as demonstrated by morphological and ultrastructural criteria. The stimulatory effect of **Compound 3f** is dose- dependent. Apoptosis induced by 10µm **Compound 3f** began after 16 hr and by 36 hr the number of apoptotic cells dramatically increases. Our results indicate that **Compound 3f** induces apoptosis in MOLT-4 cells at 10µm.

### Example 13

### In vivo toxicological screening

**Compound 3f was** also subjected to toxicological evaluation.

***Mice:*** Swiss albino male mice of about 6 weeks of age weighing 24 ± 2 g were divided in two groups as control (untreated) and treated. 6 animals were used for a particular group in an experiment. Abbreviations used for groups of mice are NC - normal control; NT - normal treated; SC - S-180 control; ST - S-180 treated.

***Vehicle and route:*** Physiological saline containing 2% Tween 80 was used for drug administration through intraperitoneal (i.p.) injection. The drug solutions were prepared daily just prior to the injection.

***Tumor:*** Sarcoma-180 (S-180) was obtained from National Centre for Cell Sciences, Pune (India). Mice were inoculated with 5.0 x 10⁶ viable cells/mouse at an injection volume of 0.2 ml on day 0 thus having 10⁶ cells/mouse for *in vivo* study.

***Dose:*** The dose 60 mg/kg was administered in normal and S-180 bearing mice from day 1 to 7. Various parameters were measured sequentially after 48 hr (day 9) for noting immediate effects, after 168 hr (day 14) for intermediate effects and after 288 hr (day 19) for late effects.

***Statistical analysis:*** Values were recorded as mean x ± S.E.M. Experimental results were analyzed by Student's t-test. P < 0.05 was considered as the level of significance for values obtained for treated groups, compared with the tumor control groups.

***Body weight:*** In case of **Compound 3f,** no adverse effects in respect of body weights were noted in treated mice. Furthermore, toxic symptoms were not observed externally in animals, in terms of their general appearance in respect of their skin and hair texture and in their behavioral pattern in respect of food and water intake and activity. The internal organs like heart, spleen, liver and kidney all retained their usual distinctive colors and appearances in the treated groups on different days as of normal control mouse. It was further noted that there were no significant changes in the weights of kidney; liver and spleen in the NT mice compared to NC mice. However, there was an increase in the weight of spleen in the ST mice compared to NC mice.

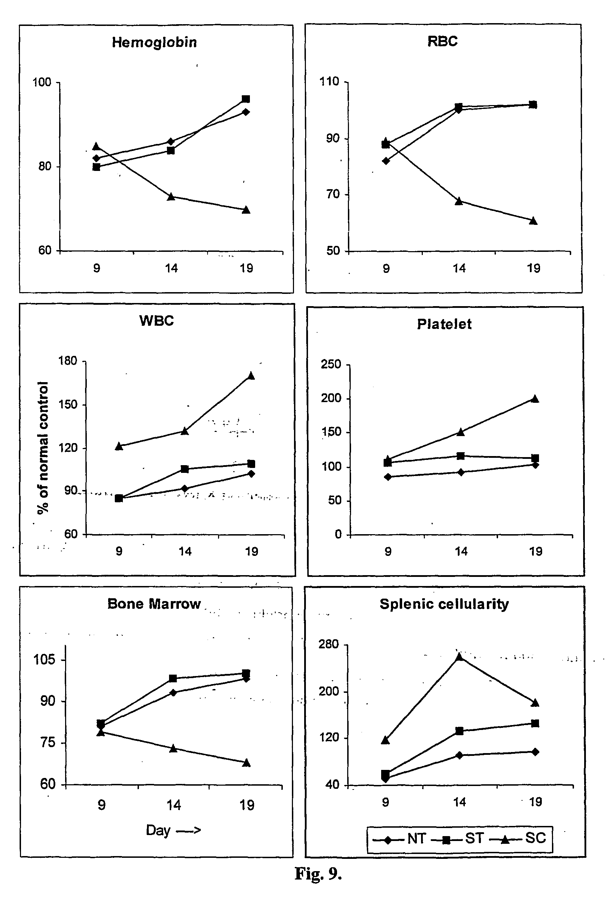

***Hematological parameters:*** Blood samples were collected by cardiac puncture from recently sacrificed animals under exposure to pentothal sodium for counting erythrocytes, thrombocytes or leukocytes in an improved Neubauer bright field counting chamber by standard procedures using freshly prepared RBC and WBC counting fluids. Hemoglobin concentrations were measured by the standard method. Sera were obtained from blood samples collected as above. Results are presented in Fig. 9.

***Total count of femoral bone marrow cellularity*:** Femoral marrow cells were obtained as per standard procedure from mice sacrificed as stated above. The ends of the femur bone were snipped open immediately thereafter with scissors and the marrow plug were flushed out by forcefully injecting cold HBSS (Ca⁺⁺ and Mg⁺⁺ free) through the bone cavity by inserting a 22 gauze needle. The marrow plug was dissociated into single cell suspension by repeatedly passing this suspension through 22 gauze needles and the total volume was measured. Total number of nucleated cells per femur was counted in a hemocytometer after treating the cells with 2% glacial acetic acid in order to lyse the mature non-nucleated erythrocytes. All the operations were carried out aseptically. Results are presented in Fig. 9.

***Total count of spleenic cellularity:*** After removing from sacrificed mouse, the whole spleen was minced in cold HBSS and the resultant mixture was passed repeatedly through 22 gauze needles to make a single cell suspension. The total number of nucleated cells in the spleen was counted in a haemocytometer after treatment with 2% glacial acetic acid. Results are presented in Fig. 9.

To study the hematological changes associated with drug application, four parameters - (a) hemoglobin level (b) erythrocyte count (c) leukocyte count and (d) platelet counts were determined in the peripheral blood of NT, ST and SC groups. Femoral bone marrow and spleenic cellularities were also determined. The detailed data obtained for each parameter in these groups for Compound 3f were expressed in Fig. 9 as the percent control of the respective values obtained for NC mice [Hemoglobin -14.5 gm/dl; RBC - 7.7 x 10⁶ /µl; WBC - 8.2 x 10³ /µl; platelets - 6.9 x 10⁵ /µl; femoral bone marrow cell count -12.2 x 10⁶ /µl; spleenic cell count -15.2 x 10⁷/ µl]. From Fig. 8, it was noted that there were about 15 - 18% decrease in the hemoglobin levels and erythrocyte counts in the NT and ST groups on day 9, which gradually tends to reach the NC value at a later stage. WBC total count results revealed there was no significant decrease or increase in the treated groups. Platelet counts showed that neither thrombocytosis nor thrombocytopenia occurred in the treated groups except slight decrease (15%) in NT group on day 9. It was found that there was slight decrease (20%) in the femoral marrow cell count in the treated groups, which was recovered by day 14. Hypospleenic cellularity was noted on day 9 in the treated groups (decrease by 42-48%), which reached normal count within day 19 (Fig. 9).

Literature survey reveals that tumorigenesis and its progression has been accompanied with the following changes compared to normalcy: (1) gradual decrease in hemoglobin content, erythrocyte count and bone marrow cellularity (2) gradual increase in leucocytes, thrombocytes and spleenic cellularity which was observed in SC mice. Slightly decreased counts in some parameters in the treated groups on day 9 were soon elevated to the normal values within day 19. All these results indicated that Compound 3f did not adversely affect haematopoiesis at its optimum dose.

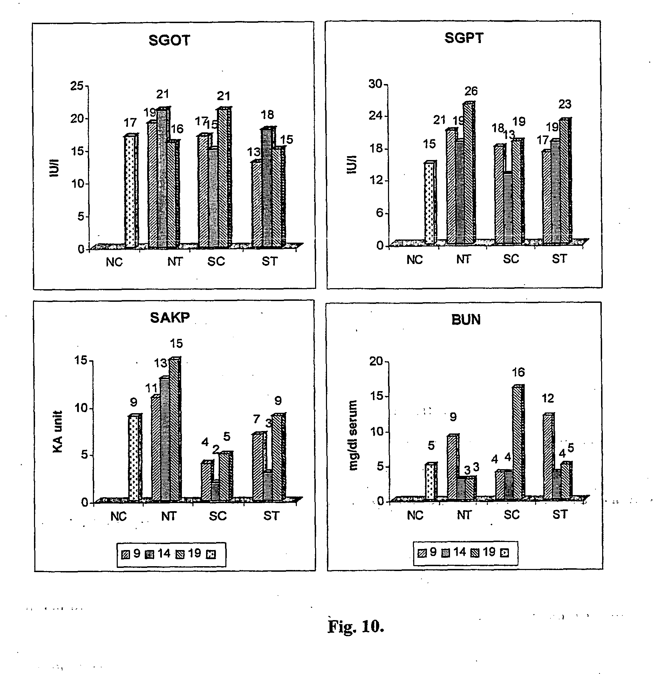

***Biochemical parameters:*** Standard methods and reagents were used to measure the serum alkaline phosphatase (SAKP), glutamic oxaloacetic transaminase (SCOT), glutamic pyruvic transaminase (SGPT), and blood urea nitrogen (BUN) levels in the collected sera [Fig. 10].

For evaluating drug-induced hepatotoxicity and nephrotoxicity, SAKP, SGPT, SGOT and BUN values obtained for the treated groups were compared with those of NC mice (respective values are 16.0 IU/l, 6.5 IU/l, 6.6 KA unit and 12.0 mg/dl). It is worth noting that all the values remained within normal range in the treated groups (Fig. 10) in case of **Compound 3f.** It is well known that there are significant elevations in the levels of SAKP and SGPT in liver diseases and damages caused by a number of agents. An increase in the SGOT level is observed in patients with cardiac damage due to myocardial infarction and with liver disorders. An increase in the BUN level is noted in cases of renal diseases and damage (BUN level > 30.0 mg/dl is considered significant for toxicity). Since the values remained within the normal range in treated groups, **Compound 3f has** not displayed hepatotoxicity or nephrotoxicity at its optimum dose.

### Histopathological studies of liver and kidney:

After injecting mice with the above stated optimum dose of Compound 3f (60mg/kg body weight for day 1-7 treatment through i.p. route), mice were sacrificed on day 9, 14 and 19. The liver and kidney were collected from those mice and histological sections were done after staining with hematoxylin & eosin [H & E] as per standard methods. Liver and kidney were also collected from normal control mice and S-180 tumor bearing mice on day 9, 14 and 19 for comparative study.

### Histology of liver:

### A. Control groups

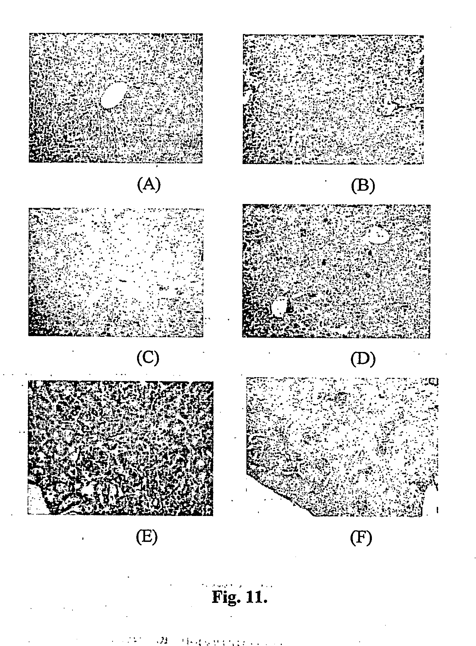

a) Hematoxylin & eosin [H & E] - stained liver section of Normal Control (NC) mouse showed as usual; a distinct central vein within a lobule and a portal area consisting of interlobular branch of portal vein, bile duct, branch of a hepatic artery & interlobular septum were visible (Fig: 11A).
b) Histology of liver of S-180 bearing (14 day) mouse showed hyperactive Kupffer cells and lymphocyte infiltration in areas adjacent to central vein as well as hepatic parenchyma [H & E - stained, x 200 or 400] (Fig. 11B).

### B. Treated groups

a) Histology of normal Swiss mice treated with **Compound 3f** (NT) showed normal architecture of hepatic lobes and hepatocytes on day 9, 14 and 19 [i.e. 48, 168 and 288 hr after completion of treatment] suggesting little or no hepatotoxicity of the **compound 3f** in normal mouse [Figures not presented].
b) Histology of liver of S-180 treated mice with **Compound 3f** showed hyperactive Kupffer cell and lymphocyte infiltration on day 9 suggesting mild inflammatory changes in liver.
c) On day 14, histology of the liver of S-180 treated mice with Compound 3f revealed infiltration of lymphocytes in lower numbers relative to S-180 control mice. Activation of Kupffer cells was enhanced compared to day 9 and focal necrosis was observed. However there was no distention or obliteration of the central vein, portal vein, hepatic sinusoids or the bile ducts [H & E - stained, x 200 or 400] (Fig. 11C).
d) On day 19, Kupffer cells activity returned to pre-treatment level and there were no trace of lymphocyte infiltration. Hepatocytes were normal and regularly arranged within the hepatic parenchyma, suggesting hepatic recovery. No fibrosis was observed in the liver of treated mice during the entire period of observation [H & E - stained, x 200 or 400] (Fig. 11D).

Thus **Compound 3f** has only mild hepatotoxicity in S-180 bearing mice when used in the present dose schedule. More importantly, the recovery is rapid and liver histology returned to normalcy within day 19.

### Histology of kidney:

Photomicrographs of kidney of a) normal treated (NT) (Fig. 11E) and b) S-180 treated (ST) mice showed normal histology (Fig. 11F). The cortex contained well-disposed renal corpuscles (i.e. intact Bowman's capsule & glomerulus), convoluted tubules and medullary rays. The inner medulla region contained the Henle's loop and the collecting tubules [H & E - stained, x200 or 400].

Thus Compound 3f [or its metabolite(s)] at its optimum dose does not possess nephrotoxicity. This observation was further supported by serum BUN and creatinine assay.

### Example 14

***³H Thymidine and** ³**H**-**Uridine incorporation in S-180 cells in vitro:*** ³H-thymidine (specific activity 1.0 mCi/ml) and ³H-uridine (specific activity 1.0 mCi/ml) were obtained from Board of Radiation and Isotope Technology, Mumbai, India. Radioactivity was measured in a liquid scintillation counter. Tumor cells aspirated from a mouse bearing S-180 for 7 days were washed twice in Hank's balanced salt solutions and re-suspended in RPMI-1640 medium supplemented with 10% heat inactivated fetal calf serum, streptomycin (100 µg/ml) and penicillin (100 units/ml). Cell suspensions taken in glass tubes were made and adjusted in such a fashion so that the final cell count became 1 x 10⁶/100 µl after the addition of ³H-thymidine or ³H-uridine (activity 10 µCi each) dissolved in 10 µl sterile saline and test compound at 8 µM concentration based on the concentration of Mitonafide reported. The tubes were incubated at 37°C for 30 and 60 min. Cell viability assessed by trypan blue dye exclusion test was of the order of 95%. The cells were harvested at 0, 30 and 60 min of incubation and absorbed onto 25-mm discs of Whatman 3MM filter papers. The dried discs were washed twice with ice-cold 10% TCA followed by with absolute alcohol. The discs were then dried in air, placed in scintillation vials containing scintillation fluid and the radioactivity was counted. Experiments were carried out in triplicate.

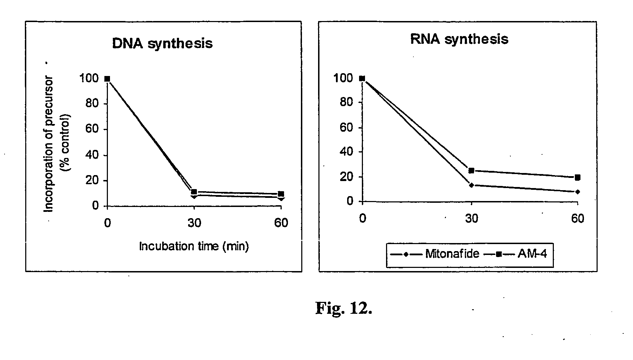

For background counts, filter papers were soaked with 10 µl of 10 µCi of ³H-thymidine or ³H-uridine, processed as described above and the radioactivity counted. Actual incorporations of the isotopes in the drug-treated groups were calculated by subtracting the background count from the observed counts. Results were expressed as percentage of the incorporation in the appropriate control without drug (Fig. 12).

Since Compound 3f has structural resemblance with Mitonafide, studies were conducted to ascertain whether drug-induced tumor growth inhibition was also due to the inhibitory effect of this compound on nucleic acid synthesis apart from its apoptotic property. Hence ³H-thymidine and ³H-uridine uptake by S-180 cells harvested from untreated mouse were evaluated after treating the cells *in vitro.* The untreated S-180 cells demonstrated an almost linear pattern of ³H-thymidine and ³H-uridine uptake over a period of 60 min incubation. Simultaneous exposure of tumor cells to compound 3f at the concentration of 8 µM resulted in gradual and marked inhibition of ³H-thymidine and ³H-uridine uptake comparable to that of Mitonafide at the same concentration (8 µM). After 1 hr incubation time, with respect to control Mitonafide inhibited 93% of DNA synthesis inhibition while respective values for Compound 3f were 90%. It was also noted that Compound 3f and Mitonafide have exhibited greater inhibitory effect on DNA synthesis compared to RNA synthesis. The respective values for Mitonafide and **Compound 3f** are as the following 92% and 80% in respect of RNA synthesis (Fig. 12).

### Brief description of figures:

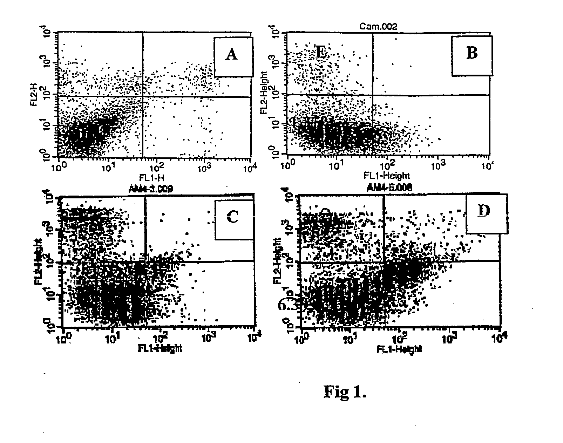

Fig 1. Analysis of apoptosis induced by Compounds in MOLT-4 cells by Flow cytometry using annexin V-FITC and PI. Quadrant analysis of fluorescence intensity of gated cells in FL-1 and FL-2 channels was from 10,000 events. Cells that were annexin⁺ /PI⁻ [LR] and annexin⁺ /PI⁺ [UR] were considered as early apoptotic and late apoptotic respectively and added to express total apoptotic activity. Cells in the upper left [UL] quadrant are considered as necrotic. A: control (1.35%); B: camptothecin (5µM, 8.83%); C - D: Compound 3f (3, 5 µg/ml; 7.43% and 18.44%).
Fig. 2. Caspase-3 activity of **Compound 3f (AM-4)** in MOLT-4 cells
**Fig. 3****.** Caspase-6 Activity of **Compound 3f (AM-4)** in MOLT-4 cells
**Fig. 4****.** Detection of sub Gl fraction (% apoptosis) in DNA Cell **Cycle** induced by Compound **3f (AM-4)** treated **MOLT-4** cells by Flow Cytometry. A: Control; B: Camptothecin (5 µM); C & D: **Compound 3f** (3 & 5 µg/ml). Values in brackets indicate concentration.
**Fig 5****.** Analysis of apoptosis induced by Compounds in HL-60 cells by Flow cytometry using annexin V-FITC and PI. Quadrant analysis of fluorescence intensity of gated cells in FL-1 and FL-2 channels was from 10,000 events. A: Stained control (2.54%); B & C: camptothecin (5 µM, 13.3%); (10µM, 15.8%); D & E: **Compound 3f** (5 µM, 47.9%) & (10µM, 92.5%); F & G: **Compound 3b** (5 µM, 9.7%) & (10µM, 48.8%). The values in brackets indicate concentration and apoptotic cell population.
**Fig. 6****.** Sequential changes in ascites cell count and fluid measurement in S-180 bearing mice after treatment with **compound 3f** from day 1-7.
**Fig. 7****.** Light microscopy of **semithin sections** of control (A), **Compound 3f** treated (B) **MOLT-4** cells. A high rate of apoptosis appears **after** incubation with **Compound 3f** at 36 hrs at 10 µM**.** Among the apoptotic cells, most of these show marginlisation of chromatin material (arrow), numerous vacuolized cells (V) can also be detected. The control cells (A) showing comparable normal morphology having large sized nuclei (N) with nucleoi. (Mag. 900X).
**Fig. 8****.** TEM of control and **Compound 3f** treated (10µM for 36 hrs) MOLT-4 cells showing internal ultra structure. The control cells show nucleus (N) with finely dispersed chromatin material, nuclear pore (NP) and a nucleolus (Nu). The mitochondria with cristae (MC) and ribosomes (R) are seen (Fig. A-C). The treatment causes chromatin marginalisation (CM), condensation of the nucleus (CN), and vacuolisation (V) in the cytoplasm (Fig. D-E).
**Fig. 9****.** Sequential changes in hematological parameters, femoral bone marrow and spleenic cellularity in normal and S-180 bearing mice after treatment with **compound 3f** from day 1-7
**Fig. 10****.** Sequential changes in biochemical parameters in normal and S-180 bearing mice after treatment with **compound 3f** from day 1-7.
**Fig. 11****.** Photomicrograph of hematoxylin & eosin [H & E] - stained sections. A. Liver of Normal Control (NC) mouse; B. Liver of S-180 bearing (SC, day 14) mouse; C. Liver of AM-4 treated S-180 bearing (ST, day 14) mouse. D. Liver of AM-4 treated S-180 bearing (ST, day 19) mouse. [A-D magnification x 200]. E. Kidney of Normal Control (NC) mouse; F. Kidney of AM-4 treated Normal (NT, day 9) mouse; [E-F magnification x 400].
**Fig. 12****.** Effects of compounds **(Compound 3f** and Mitonafide) on the synthesis of DNA and RNA in S-180 tumor cells.

**Table 1. Physicochemical data of the N-(2-chloroethyl) and N-(3-chloropropyl) naphthalimide compounds (3a-g)**

| **Compd No.** | **Status** | **R** | **n** | **Melting point (°C)** | **Yield %** | **Mol. formula** | **Mol. wt** |
|---|---|---|---|---|---|---|---|
| **3a** | **unknown** | 6-Br | 1 | 193-194 | 60 | C₁₄H₉BrClNO₂ | 338.5 |
| **3b** | **unknown** | 6-NO₂ | 1 | 182-184 | 69 | C₁₄H₉ClN₂O₄ | 304.5 |
| **3c** | **unknown** | 5-NO₂ | 1 | 203-205 | 83 | C₁₄H₉ClN₂O₄ | 304.5 |
| **3d** | **unknown** | 6-Br | 2 | 132-134 | 75 | C₁₅H₁₁BrClNO₂ | 352.5 |
| **3e** | **unknown** | 6-Cl | 2 | 115-117 | 89 | C₁₅H₁₁Cl₂NO₂ | 308 |
| **3f** | **unknown** | 6-NO₂ | 2 | 155-156 | 62 | C₁₅H₁₁ClN₂O₄ | 318.5 |
| **3g** | **unknown** | 5-NO₂ | 2 | 175-177 | 64 | C₁₅H₁₁ClN₂O₄ | 318.5 |

**Table 2.¹HNMR spectral data of compounds 3a-g**

| **Compd. No.** | **¹H NMR *** |
|---|---|
| **3a** | 3.86 (t, 2H, CH₂Cl), 438 [t, 2H, CH₂N(CO)₂)], 8.0 (t, 1H, arom H), 8.24 (d, 1H, arom. H), 8.36 (d, 1H, arom. H). 8.57 (m, 2H, arom. H). |
| **3b** | 3.86 (t, 2H, CH₂Cl), 4.59 [t, 2H, CH₂N(CO)₂)], 8.0 (t, 1H, arom H), 8.43 (d, 1H, arom. H), 8.75 (m, 2H, arom. H). 8.87 (d, 1H, arom. H). |
| **3c** | 3.87 (t, 2H, CH₂Cl), 4.41 [t, 2H, CH₂N(CO)₂)], 8.07 (t, 2H, arom H), 8.71 (d, 1H, arom. H), 8.81 (d, 1H, arom. H), 8.96 (s, 1H, arom. H). |
| **3d** | 2.25 (m, 2H, CH₂), 3.65 (t, 2H, CH₂Cl), 4.33 [t, 2H, CH₂N(CO)₂)], 7.88 (m, 1H, arom.H), 8.03 (d, 1H, arom. H), 8.43 (d, 1H, arom. H), 8.60 (d, 1H, arom. H), 8.67 (d, 1H, arom. H). |
| **3e** | 2.24 (m, 2H, CH₂), 3.65 (t, 2H, CH₂Cl), 4.33 [t, 2H, CH₂N(CO)₂)], 7.86 (m, 2H, arom.H), 8.51 (d, 1H, arom. H), 8.62 (m, 2H, arom. H). |
| **3f** | 2.25 (m, 2H, CH₂), 3.66 (t, 2H, CH₂Cl), 4.36 [t, 2H, CH₂N(CO)₂)], 8.00 (t, 1H, arom.H), 8.40 (d, 1H, arom. H), 8.73 (m, 2H, arom. H), 8.86 (d, 1H, arom. H). |
| **3g** | 2.25 (m, 2H, CH₂), 3.66 (t, 2H, CH₂Cl), 4.37 [t, 2H, CH₂N(CO)₂)], 7.96 (t, 1H, arom.H), 8.46 (d, 1H, arom. H), 8.80 (d, 1H, arom.H), 9.15 (s, 1H, arom. H), 9.32 (s, 1H, arom. H). |

**Table 3. HPLC analytical data for compounds 3a-g**

| **Compound No.** | **R** | **Retention time (in min)** |
|---|---|---|
| | | Flow solvent* |
| **3a** | 6-Br | 9.2 |
| **3b** | 6-NO₂ | 6.3 |
| **3c** | 5-NO₂ | 4.9 |
| **3d** | 6-Br | 11.5 |
| **3e** | 6-Cl | 10.3 |
| **3f** | 6-NO₂ | 7.6 |
| **3g** | 5-NO₂ | 6.4 |

| | | |
|---|---|---|
| *Methanol: water (75: 25, v/v) | | |

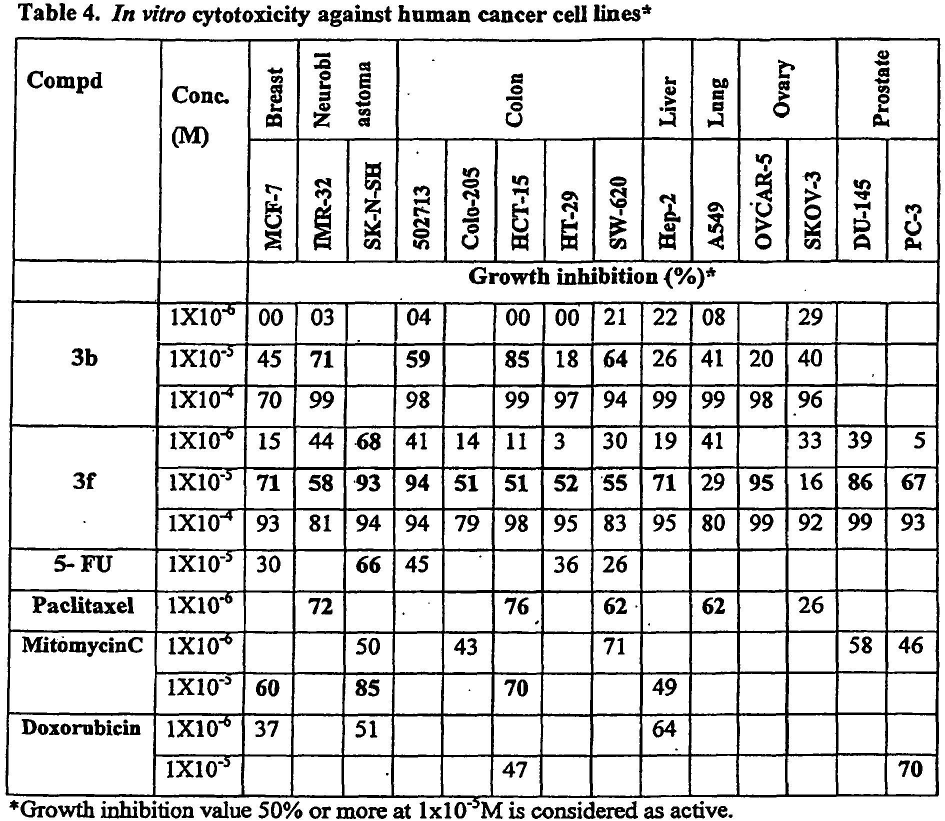

**Table 5. In vitro cytotoxicity against human cancer cell lines****

| **Compd** | **IC₅₀ value (µM)** | |
|---|---|---|
| | **Leukemia HL-60** | **Lymphoma U-937** |
| **3b** | **0.8** | **0.66** |
| **3f** | **0.3** | **1.95** |
| Cis-Platinum | 7.0 | 3.2 |
| BCNU | 30.5 | 12.3 |
| 5-FU | 266 | 4.7 |
| Hydroxyurea | 204 | 115 |

| | | |
|---|---|---|
| **IC₅₀ value < 10µM is considered as active. BCNU -Bis (2-chloroethyl- nitrosourea) | | |

**Table 6. In vivo anticancer activity for Compounds 3**

| **Compd.** | **Tumor System** | **Expt. No.** | **Dose (mg/kg)** | **No. of injns.** | **Injection days** | **MST (days)** | **Survivors > 60 days** | **T/C %** |
|---|---|---|---|---|---|---|---|---|
| | **S-180** | | 80 | 7 | 1-7 | 23.0 | - | 115 |
| **3b** | | 1 | 40 | 7 | 1-7 | 20.5 | - | 102 |
| | | | Control | | | 20.0 | - | 100 |
| | | | 60 | 7 | 1-7 | 29.0 | - | 138 |
| **3f** | | 2 | 30 | 7 | 1-7 | 25.0 | - | 116 |
| | | | 60 | 7 | 5-11 | 21.0 | - | 98 |
| | | | Control | | | 21.5 | - | 100 |
| | | | 60 | 7 | 1-7 | 24.0 | - | 129 |
| **3g** | | 3 | 30 | 7 | 1-7 | 25.5 | - | 106 |
| | | | 60 | 7 | 5-11 | 25.0 | - | 104 |
| | | | Control | | | 24.0 | - | 100 |
| 5-FU | | 4 | 20 | 7 | 1-7 | 53.5 | 3 | 233 |
| | | | Control | | | 23.0 | - | 100 |
| Cyclophosphamide | | 5 | 6 | 7 | 1-7 | 33.0 | - | 143 |
| | | | Control | | | 23.0 | - | 100 |
| | | | 1 | 7 | 1-7 | 25.0 | - | 128 |
| Mitonafide | | 6 | 0.5 | 7 | 1-7 | 36.5 | 1 | 187 |
| | | | Control | | | 19.5 | - | 100 |
| | | | 60 | 7 | 1-7 | 34.0 | - | 189 |
| **3f** | EAC | 7 | Control | | | 18.0 | - | 100 |

| | | | | | | | | |
|---|---|---|---|---|---|---|---|---|
| % T/C value > 125 is considered as significant | | | | | | | | |

## Claims

1. Novel substituted 1H-benz[de]isoquinoline-1,3-diones of general formula 3 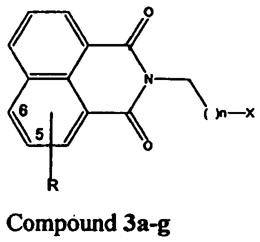 wherein R is selected from halogen, nitro; n = 1 or 2; X = Cl, such that when n=1, R=6-Br or 6-NO₂ or 5-NO₂ and when n=2, R=6-Br or 6-Cl or 6-NO₂ or 5-NO₂ or pharmaceutically acceptable salt thereof.

2. A compound as claimed in claim 1, wherein the representative compounds of the general formula 3 comprising;
(a) 6-Bromo-2-[2-(Chloroethyl)]-1H-benz[de]isoquinoline-1,3-dione
(b) 6-Nitro-2-[2-(Chloroethyl)]-1H-benz[de]isoquinoline-1,3-dione
(c) 5-Nitro-2-[2-(Chloroethyl)]-1H-benz[de]isoquinoline-1,3-dione
(d) 6-Bromo-2-[3-(Chloropropyl)]-1H-benz[de]isoquinoline-1,3-dione
(e) 6-Chloro-2-[3-(Chloropropyl)]-1H-benz[de]isoquinoline-1,3-dione
(f) 6-Nitro-2-[3-(Chloropropyl)]-1H-benz[de]isoquinoline-1,3-dione
(g) 5-Nitro-2-[3-(Chloropropyl)]-1H-benz[de]isoquinoline-1,3-dione

3. A process for preparation of novel substituted 1H-benz[de]isoquinoline-1,3-diones of general formula 3, 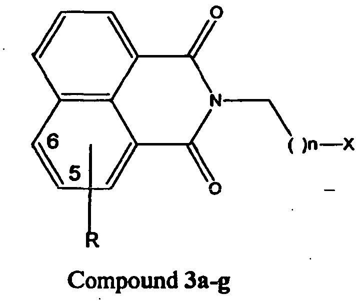 wherein R is selected from halogen and nitro; n = 1 or 2; X = Cl, such that when n=1, R=6-Br or 6-NO₂ or 5-N02 and when n=2, R=6-Br or 6-Cl or 6-NO₂ or 5-NO₂,
wherein the step comprises; reacting substituted naphthalimide hydroxy derivative of general formula **2a-g** 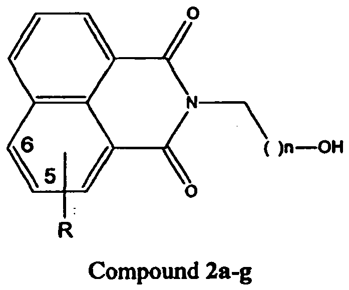 wherein R is 6-Br or 6-Cl or 6-NO₂ or 5-NO₂ and n=1 or 2, such that when n=1, R is 6-Br, 6-NO₂, or 5-NO₂ and when n=2, R=6-Br or 6-Cl or 6-NO₂ or 5-NO₂ with POCl₃ under inert and dry atmosphere at a temperature ranging between 80-90°C for a period ranging between 1 to 2 hr, evaporating excess POCl₃ to obtain the resultant and followed by purification by standard methods to obtain the desired compound.

4. A process as claimed in claim 3, wherein:
• the reaction is carried out under inert atmosphere using an inert gas;
• the ratio of POCl₃ to the compound is in the range of 2 to 3 molar times;
• purification of the compound is carried out by chromatographic method; or
• the reaction is carried out at a temperature ranging between 80 to 90°C.

5. A pharmaceutical composition comprising an effective amount of a compound of general formula 3, or salts thereof, wherein R is selected from halogen, nitro; n = 1 or 2; X = Cl such that when n=1, R=6-Br or 6-NO₂ or 5-NO₂ and when n=2, R=6-Br or 6-Cl or 6-NO₂ or 5-NO₂ along with a pharmaceutical carrier, diluents, additives or a lubricant.

6. The pharmaceutical composition of claim 5 for use in the treatment of cancer.

7. Novel substituted 1H-benz[de]isoquinoline-1, 3-diones of general formula 3 according to claim 1 of the general formula of compound 3b and 3f 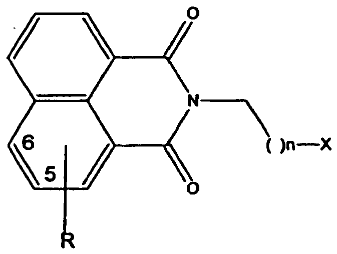 3b; wherein R = 6-NO₂; X = Cl; n = 1
3f ; wherein R = 6-NO₂; X = Cl; n = 2.

8. A pharmaceutical composition comprising an effective amount of a compound of claim 7 of general formula 3b and 3f, or salts thereof, wherein R is 6-nitro; n = 1 or 2; X = Cl, along with a pharmaceutical carrier, diluents, additives or a lubricant 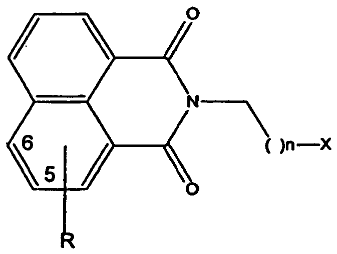 3b; wherein R = 6-NO₂; X = Cl; n = 1 3f ; wherein R = 6-NO₂; X = Cl; n = 2.

9. The pharmaceutical composition as claimed in claim 8 for use in the treatment of cancer.

10. The pharmaceutical composition as claimed in claim 8 for use according to claim 9, wherein the carrier used is selected from the group consisting of cellulosic suspending agents, polymeric suspending agents, xanthan gum, silicone dioxide and mixtures thereof.

11. The pharmaceutical composition as claimed in claim 8 for use according to claim 9, wherein the diluent used is selected from the group consisting of lactose, dicalcium phosphate, mannitol and corn starch.

12. The pharmaceutical composition as claimed in claim 8 for use according to claim 9, wherein the additive used is selected from the group consisting of sodium citrate, calcium carbonate, dicalcium phosphate together with various additional substances such as starch, gelatin.

13. The pharmaceutical composition as claimed in claim 8 for use according to claim 9, wherein the lubricant used is selected from the group consisting of magnesium stearate, calcium stearate, glycerol monostearate, hydrogenated caster oil / hydrogenated vegetable oil, mineral oil, polyethylene glycol, sodium lauryl sulphate,

14. The pharmaceutical composition as claimed in claim 8 for use according to claim 9, wherein:
• the composition is to be used at doses ranging between 30 to 80 mg/kg;
• the composition is to be administered by oral, intravenous, intraperitoneal, intramuscular, subcutaneous, intrauterine routes; or
• the composition is to be used in the form of tablet, injectable, syrup, capsule.

15. A composition as claimed in claim 8 for use according to claim 9, wherein the salt of the compounds of general formula comprised therein is any pharmaceutically acceptable salt; preferably is hydrochloride, hydrobromide, or a salt of acetic acid, mesylic acid.

## Patentansprüche

1. Neue substituierte 1 H-benz[de]isoquinolin-1,3-dione der allgemeinen Formel 3 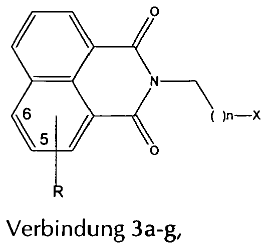 wobei R ausgewählt ist aus Halogen, Nitro; n=1 oder 2; X=Cl, so dass, wenn n=1, R=6-Br oder 6-NO₂ oder 5-NO₂ und wenn n=2, R=6-Br oder 6-Cl oder 6-NO₂ oder 5-NO₂ oder ein pharmazeutisch akzeptables Salz davon.

2. Verbindung, wie in Anspruch 1 beansprucht, wobei die repräsentativen Verbindungen der allgemeinen Formel 3 umfassen;
(a) 6-Bromo-2-[2-(Chloroethyl)]-1H-benz[de]isoquinolin-1,3-dion
(b) 6-Nitro-2-[2-(Chloroethyl)]-1H-benz[de]isoquinolin-1,3-dion
(c) 5-Nitro-2-[2-(Chloroethyl)]-1H-benz[de]isoquinolin-1,3-dion
(d) 6-Bromo-2-[3-(Chloropropyl)]-1H-benz[de]isoquinolin-1,3-dion
(e) 6-Chloro-2-[3-(Chloropropyl)]-1H-benz[de]isoquinolin-1,3-dion
(f) 6-Nitro-2-[3-(Chloropropyl)]-1H-benz[de]isoquinolin-1,3-dion
(g) 5-Nitro-2-[3-(Chloropropyl)]-1H-benz[de]isoquinolin-1,3-dion.

3. Verfahren zur Herstellung neuer substituierter 1H-benz[de]isoquinolin-1,3-dione der allgemeinen Formel 3, 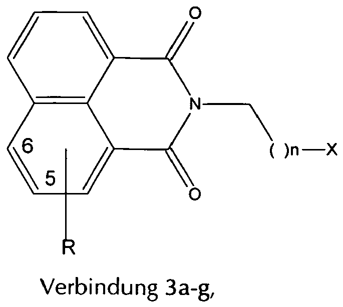 wobei R ausgewählt ist aus Halogen und Nitro; n=1 oder 2; X=Cl, so dass, wenn n=1, R=6-Br oder 6-NO₂ oder 5-NO₂ und, wenn n=2, R=6-Br oder 6-Cl oder 6-NO₂ oder 5-NO₂,
wobei der Schritt umfasst; Reagieren des substituierten Naphthalimid-Hydroxyderivats der allgemeinen Formel 2a-g 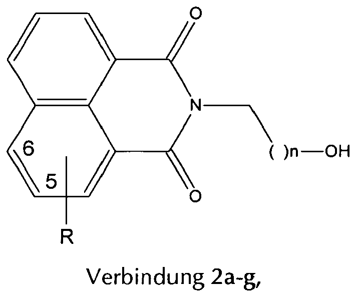 wobei R 6-Br oder 6-Cl oder 6-NO₂ oder 5-NO₂ ist und n=1 oder 2 ist, so dass, wenn n=1, R 6-Br, 6-NO₂ oder 5-NO₂ ist und wenn n=2, R=6-Br oder 6-Cl oder 6-NO₂ oder 5-NO₂ mit POCl₃ unter inerter und trockener Atmosphäre bei einer Temperatur im Bereich zwischen 80-90°C für einen Zeitraum zwischen 1 und 2 Stunden, Evaporieren des überschüssigen POCl₃ zum Erhalt des Produkts, gefolgt von einer Reinigung durch Standardverfahren zum Erhalt der gewünschten Verbindung.

4. Verfahren nach Anspruch 3, wobei:
• die Reaktion unter inerter Atmosphäre unter Verwendung eines inerten Gases ausgeführt wird;
• das Verhältnis von POCl₃ zur Verbindung im Bereich zwischen dem 2- bis 3-molarfachen ist;
• Reinigung der Verbindung durch ein chromatographisches Verfahren ausgeführt wird; oder
• die Reaktion bei einer Temperatur im Bereich zwischen 80 und 90°C ausgeführt wird.

5. Pharmazeutische Zusammensetzung, umfassend eine wirksame Menge einer Verbindung der allgemeinen Formel 3 oder Salzen davon, wobei R ausgewählt ist aus Halogen, Nitro; n=1 oder 2; X=Cl, so dass, wenn n=1, R=6-Br oder 6-NO₂ oder 5-NO₂ und wenn n=2, R=6-Br oder 6-Cl oder 6-NO₂ oder 5-NO₂, gemeinsam mit einem pharmazeutischen Träger, Streckmitteln, Additiven oder einem Schmiermittel.

6. Pharmazeutische Zusammensetzung nach Anspruch 5 zur Verwendung in der Behandlung von Krebs.

7. Neue substituierte 1H-benz[de]isoquinolin-1,3-dione der allgemeinen Formel 3
nach Anspruch 1 der allgemeinen Formel von Verbindung 3b und 3f 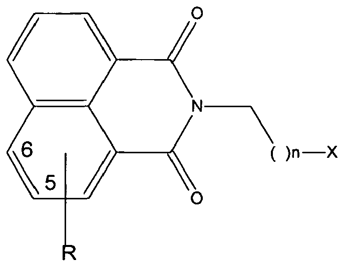 3b; wobei R=6-NO₂; X=Cl; n=1
3f; wobei R=6-NO₂; X=Cl; n=2

8. Pharmazeutische Zusammensetzung, umfassend eine wirksame Menge einer Verbindung nach Anspruch 7 der allgemeinen Formel 3b und 3f oder Salzen davon, wobei R 6-Nitro ist; n=1 oder 2; X=Cl, gemeinsam mit einem pharmazeutischen Träger, Streckmitteln, Additiven oder einem Schmiermittel 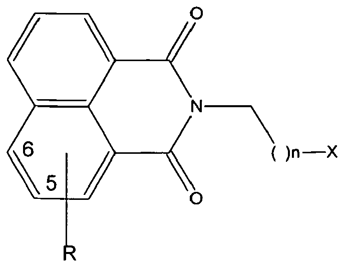 3b; wobei R=6-NO₂; X=Cl; n=1
3f; wobei R=6-NO₂; X=Cl; n=2.

9. Pharmazeutische Zusammensetzung nach Anspruch 8 zur Verwendung in der Behandlung von Krebs.

10. Pharmazeutische Zusammensetzung nach Anspruch 8 zur Verwendung nach Anspruch 9, wobei der verwendete Träger ausgewählt ist aus der Gruppe bestehend aus celluloseartigen suspendierenden Agentien, polymerartigen suspendierenden Agentien, Xanthangummi, Silicondioxyd und Gemischen davon.

11. Pharmazeutische Zusammensetzung nach Anspruch 8 zur Verwendung nach Anspruch 9, wobei das verwendete Streckmittel ausgewählt ist aus der Gruppe bestehend aus Laktose, Dicalciumphosphat, Mannitol oder Maisstärke.

12. Pharmazeutische Zusammensetzung nach Anspruch 8 zur Verwendung nach Anspruch 9, wobei das verwendete Additiv ausgewählt ist aus der Gruppe bestehend aus Natriumcitrat, Calciumcarbonat, Dicalciumphosphat zusammen mit verschiedenen zusätzlichen Substanzen, wie beispielsweise Stärke, Gelatine.

13. Pharmazeutische Zusammensetzung nach Anspruch 8 zur Verwendung nach Anspruch 9, wobei das verwendete Schmiermittel ausgewählt ist aus der Gruppe bestehend aus Magnesiumstearat, Calciumstearat, Glycerolmonostearat, hydrogeniertes Rizinusöl/hydrogeniertes Pflanzenöl, Mineralöl, Polyethylenglykol, Natriumlaurylsulphat.

14. Pharmazeutische Zusammensetzung nach Anspruch 8 zur Verwendung nach Anspruch 9, wobei:
• die Zusammensetzung in Dosierungen zwischen 30 und 80 mg/kg zu verwenden ist;
• die Zusammensetzung durch orale, intravenöse, intraperitoneale, intramuskuläre, subkutane, intrauterine Verabreichungswege zu verabreichen ist; oder
• die Zusammensetzung in Form einer Tablette, zur Injektion, als Sirup, als Kapsel zu verwenden ist.

15. Zusammensetzung nach Anspruch 8 zur Verwendung nach Anspruch 9, wobei das Salz der Verbindungen der allgemeinen Formel 3, das darin umfasst ist, irgendein pharmazeutisch akzeptables Salz ist; vorzugweise Hydrochlorid, Hydrobromid oder ein Salz der Essigsäure, Mesylat.

## Revendications

1. Nouvelles 1H-benz[de]isoquinoléine-1,3-diones substituées de
formule générale 3 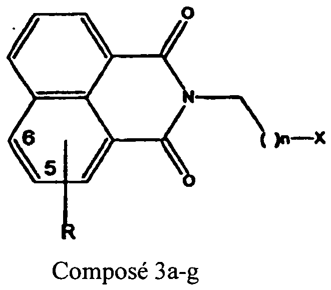 où R est choisi parmi halogène, nitro ; n = 1 ou 2 ; X = Cl, de sorte que quand n = 1, R = 6-Br ou 6-NO₂ ou 5-NO₂ et quand n = 2, R = 6-Br ou 6-Cl ou 6-NO₂ ou 5-NO₂ ou sel pharmaceutiquement acceptable de celles-ci.

2. Composé selon la revendication 1 où les composés représentatifs de formule générale 3 comprenant :
(a) la 6-bromo-2-[2-(chloroéthyl)]-1H-benz[de]isoquinoléine-1,3-dione
(b) la 6-nitro-2-[2-(chloroéthyl)]-1H-benz[de]isoquinoléine-1,3-dione
(c) la 5-nitro-2-[2-(chloroéthyl)]-1H-benz[de]isoquinoléine-1,3-dione
(d) la 6-bromo-2-[3-(chloropropyl)]-1H-benz[de]isoquinoléine-1,3-dione
(e) la 6-chloro-2-[3-(chloropropyl)]-1H-benz[de]isoquinoléine-1,3-dione
(f) la 6-nitro-2-[3-(chloropropyl)]-1H-benz[de]isoquinoléine-1,3-dione
(g) la 5-nitro-2-[3-(chloropropyl)]-1H-benz[de]isoquinoléine-1,3-dione

3. Procédé pour la préparation de nouvelles 1H-benz[de]isoquinoléine-1,3-diones substituées de formule générale 3 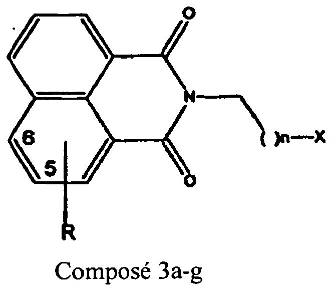 où R est choisi parmi halogène et nitro ; n = 1 ou 2 ; X = Cl, de sorte que quand n = 1, R = 6-Br ou 6-NO₂ ou 5-NO₂ et quand n = 2, R = 6-Br ou 6-Cl ou 6-NO₂ ou 5-NO₂,
où l'étape comprend : la réaction d'un dérivé hydroxy de naphtalimide substitué de formule générale 2a-g 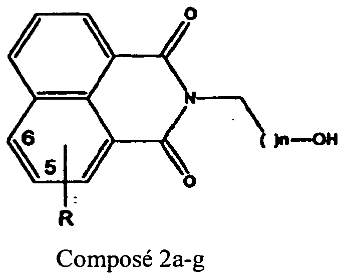 où R est 6-Br ou 6-Cl ou 6-NO₂ ou 5-NO₂ et n = 1 ou 2, de sorte que quand n = 1, R = 6-Br, 6-NO₂ ou 5-NO₂ et quand n = 2, R = 6-Br ou 6-Cl ou 6-NO₂ ou 5-NO₂ avec POCl₃ dans une atmosphère inerte et sèche à une température située entre 80-90°C pendant une durée située entre 1 et 2 h, l'évaporation de POCl₃ en excès pour obtenir le résultant et suivie par la purification par des procédés standard pour obtenir le composé souhaité.

4. Procédé selon la revendication 3 où :
• la réaction est conduite sous atmosphère inerte au moyen d'un gaz inerte ;
• le rapport de POCl3 au composé est dans la plage de 2 à 3 fois molaire ;
• la purification du composé est accomplie par un procédé chromatographique ; ou
• la réaction est conduite à une température située entre 90 et 90°C.

5. Composition pharmaceutique comprenant une quantité efficace d'un composé de formule générale 3, ou de sels de celui-ci, où R est choisi parmi halogène, nitro ; n = 1 ou 2 ; X = Cl de sorte que quand n = 1, R = 6-Br ou 6-NO₂ ou 5-NO₂ et quand n = 2, R = 6-Br ou 6-Cl ou 6-NO₂ ou 5-NO₂ avec un vecteur, des diluants, des additifs ou un lubrifiant pharmaceutiques.

6. Composition pharmaceutique selon la revendication 5 destinée à être utilisée dans le traitement du cancer.

7. Nouvelles 1H-benz[de]isoquinoléine-1,3-diones substituées de formule générale 3 selon la revendication 1 de la formule générale de composé 3b et 3f 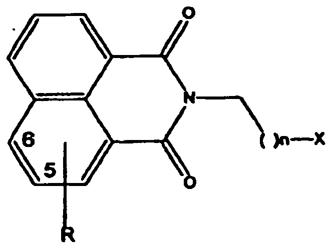 3b; où R=6-NO₂; X=Cl ; n=1
3f; où R=6-NO₂ ; X=Cl ; n=2.

8. Composition pharmaceutique comprenant une quantité efficace d'un composé selon la revendication 7 de formule générale 3b et 3f, ou de sels de celui-ci, où R est 6-nitro ; n = 1 ou 2 ; X = Cl, avec un vecteur, des diluants, des additifs ou un lubrifiant pharmaceutiques 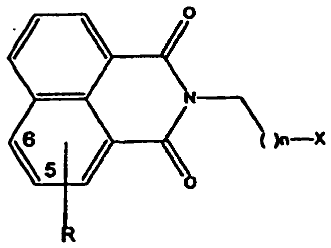 3b; où R=6-NO₂ ; X=Cl; n=1
3f; où R=6-NO₂; X=Cl; n=2.

9. Composition pharmaceutique selon la revendication 8 destinée à être utilisée dans le traitement du cancer.

10. Composition pharmaceutique selon la revendication 8 destinée à être utilisée selon la revendication 9 où le vecteur utilisé est choisi dans le groupe consistant en les agents de suspension cellulosiques, les agents de suspension polymères, la gomme de xanthane, le dioxyde de silicium et leurs mélanges.

11. Composition pharmaceutique selon la revendication 8 destinée à être utilisée selon la revendication 9 où le diluant utilisé est choisi dans le groupe consistant en le lactose, le phosphate de dicalcium, le mannitol et l'amidon de maïs.

12. Composition pharmaceutique selon la revendication 8 destinée à être utilisée selon la revendication 9 où l'additif utilisé est choisi dans le groupe consistant en le citrate de sodium, le carbonate de calcium, le phosphate de dicalcium avec différentes substances supplémentaires comme l'amidon, la gélatine.

13. Composition pharmaceutique selon la revendication 8 destinée à être utilisée selon la revendication 9 où le lubrifiant utilisé est choisi dans le groupe consistant en le stéarate de magnésium, le stéarate de calcium, le monostéarate de glycérol, l'huile de ricin hydrogénée/huile végétale hydrogénée, l'huile minérale, le polyéthylèneglycol, le laurylsulfate de sodium.

14. Composition pharmaceutique selon la revendication 8 destinée à être utilisée selon la revendication 9 où :
• la composition est destinée à être utilisée à des doses situées entre 30 et 80 mg/kg;
• la composition est destinée à être administrée par les voies orale, intraveineuse, intrapéritonéale, intramusculaire, sous-cutanée, intra-utérine ; ou
• la composition est destinée à être utilisée sous forme de comprimé, de produit injectable, de sirop, de capsule.

15. Composition pharmaceutique selon la revendication 8 destinée à être utilisée selon la revendication 9 où le sel des composés de formule générale 3 compris à l'intérieur est tout sel pharmaceutiquement acceptable ; de préférence est un chlorhydrate, un bromhydrate ou un sel de l'acide acétique, de l'acide mésylique.
